# FASCICULE DE BREVET EUROPEEN

(11) **EP 3 384 290 B1**
(45) Date de publication et mention de la délivrance du brevet: **01.04.2020**
(21) Numéro de dépôt: 16819599.8
(22) Date de dépôt: 02.12.2016
(51) Int. Cl.: G01N 33/574

(54) **METHODE DE DETECTION DE CELLULES SOUCHES CANCEREUSES**
VERFAHREN FÜR DEN NACHWEIS VON KREBSSTAMMZELLEN
METHOD FOR DETECTING CANCER STEM CELLS

(30) Priorité: 02.12.2015 FR 1561763
(43) Date de publication de la demande: 10.10.2018
(73) Titulaire: Université de Limoges, 87032 Limoges (FR)
(72) Inventeur: LACROIX, Aurélie, 87350 Panazol (FR); MATHONNET, Muriel, 87350 Panazol (FR); LALLOUE, Fabrice, 87170 Isle (FR); JAUBERTEAU, Marie-Odile, 87100 Limoges (FR)
(74) Mandataire: Grosset-Fournier, Chantal Catherine
(86) Numéro de dépôt international: PCT/FR2016/053196
(87) Numéro de publication internationale: WO 2017/093696

(56) Documents cités:
- WO-A2-2008/036419
- CAROL TUCKER-BURDEN ET AL: "Lectins Identify Glycan Biomarkers on Glioblastoma-Derived Cancer Stem Cells", STEM CELLS AND DEVELOPMENT, vol. 21, no. 13, 21 mars 2012 (2012-03-21) , - 1 septembre 2012 (2012-09-01), pages 2374-2386, XP055157421, ISSN: 1547-3287, DOI: 10.1089/scd.2011.0369
- ARISTIDES G. VAIOPOULOS ET AL: "Colorectal Cancer Stem Cells", STEM CELLS., vol. 30, no. 3, 9 janvier 2012 (2012-01-09), pages 363-371, XP055295355, US ISSN: 1066-5099, DOI: 10.1002/stem.1031 cité dans la demande

## Description

La présente invention concerne le domaine de la détection de cellules souches cancéreuses, notamment colorectales.

Le cancer colorectal (CCR) est la troisième maladie la plus fréquente dans le monde. Celle-ci comme tout cancer, peut se résumer par une prolifération cellulaire anormale dans un tissu sain, ici la muqueuse colique, provoquant l'apparition d'une masse tumorale. L'une des hypothèses émises afin d'expliquer la progression tumorale ainsi que les mécanismes de résistance et les récidives repose sur l'existence de cellules souches cancéreuses. L'échappement thérapeutique de la tumeur aux traitements radio- et chimio-thérapeutique dépend de la présence de ces cellules au sein de la tumeur. Par conséquent la détection de ces cellules dans le tissu tumoral constitue un moyen de définir le niveau d'agressivité de la tumeur. La caractérisation de biomarqueurs spécifiques des cellules souches cancéreuses est donc d'un grand intérêt diagnostique et pronostique dans le traitement du cancer. Cependant, il n'existe pas actuellement de marqueurs spécifiques des cellules souches cancéreuses (CSCs) qui permettent leur discrimination avec certitude des autres cellules tumorales.

Tucker-Burden et al. (Lectins identify glycan biomarkers on glioblastoma-derived cancer stem cells; Stem cells and development; 2012) décrit notamment l'utilisation de lectines reconnaissant le motif alpha-N-acetylgalactosamine pour la détection de cellules souches cancéreuses de glioblastome.

Aristides et al. (Concise review : colorectal cancer stem cells; Stem cells; 2012) est une revue décrivant le rôle des cellules souches cancéreuses dans le cancer colorectal et les marqueurs utilisés, tels que CD133, CD44, ALDH-1, Msi-1 ou encore Lgr-5, pour la détection de cellules souches cancéreuses.

Au vu de leur faible nombre et de l'absence de marqueurs spécifiques, les difficultés majeures pour étudier les CSCs résident dans leur isolement et leur caractérisation.

Il existe donc un besoin pour une méthode permettant de détecter les cellules souches cancéreuses colorectales dans une tumeur colorectale.

La présente invention vient combler ce besoin. Les Inventeurs de la présente invention ont identifié, en tant que marqueur des cellules souches cancéreuses colorectales, des glycanes particuliers exprimés à la surface de cette population de cellules. La reconnaissance de ces marqueurs spécifiques par des moyens appropriés, permet de détecter et de quantifier les cellules souches cancéreuses colorectales au sein d'un tissu colorectal dans lequel sont présentes des cellules non-souches non-cancéreuses, des cellules non-souches cancéreuses et des cellules souches non-cancéreuses.

Dans un premier aspect, la présente invention concerne l'utilisation d'une lectine pour la détection et/ou la quantification *in vitro* des cellules souches cancéreuses (CSC) colorectales et éventuellement d'un second moyen de marquage des cellules souches cancéreuses colorectales.

Dans un deuxième aspect, la présente invention concerne un procédé de détection et/ou de quantification des cellules souches cancéreuses colorectales dans un échantillon biologique colorectal.

Dans un troisième aspect, la présente invention concerne une méthode de diagnostic du risque de récidive et/ou de l'agressivité d'un cancer colorectal pour définir une valeur pronostique pour l'adaptation thérapeutique d'un cancer colorectal.

Dans un quatrième aspect, la présente invention concerne un kit comprenant une lectine et un second moyen de marquage des cellules souches cancéreuses colorectales, ainsi que l'utilisation dudit kit pour la détection et/ou la quantification des cellules souches cancéreuses colorectales, en particulier pour le diagnostic du risque de récidive et/ou de l'agressivité d'un cancer colorectal pour définir une valeur pronostique pour l'adaptation thérapeutique d'un cancer colorectal.

Un premier objet de la présente invention concerne donc l'utilisation d'un moyen permettant de reconnaitre spécifiquement le motif fucose α 1-2 galactose, notamment une lectine reconnaissant le motif fucose α 1-2 galactose, pour la détection et/ou la quantification des cellules souches cancéreuses colorectales.

La présente invention concerne en particulier l'utilisation d'un moyen permettant de reconnaitre spécifiquement le motif fucose α 1-2 galactose, notamment une lectine reconnaissant le motif fucose α 1-2 galactose, pour la détection et/ou la quantification *in vitro* des cellules souches cancéreuses colorectales dans un échantillon biologique colorectal.

Les cellules souches cancéreuses colorectales sont une population de cellules quiescentes, capables d'auto-renouvellement et dotées d'une résistance à de nombreuses substances utilisées en chimiothérapie. Elles sont également connues en tant que cellules initiatrices de tumeurs (CIT). Les cellules souches cancéreuses colorectales ont déjà été décrites et ont fait l'objet de revues, par exemple dans Vaiopoulos et al. S, Stem Cells 2012 (30), 363-371 et Ricci-Vitiani et al. J. Mol. Med. 2009, 87 (11), 1097-1104.

La présente invention concerne également l'utilisation, en tant que premier moyen de marquage, d'une lectine reconnaissant le motif fucose α 1-2 galactose pour la mise en œuvre d'un procédé de détection et/ou de quantification *in vitro* des cellules souches cancéreuses colorectales dans un échantillon biologique colorectal.

Au sens de la présente invention, on entend par « moyen de marquage des cellules souches cancéreuses colorectales » une substance capable de se lier spécifiquement à un marqueur exprimé à la surface des cellules souches cancéreuses colorectales. Le moyen de marquage peut en particulier être un anticorps dirigé contre un déterminant antigénique, tel qu'une glycoprotéine, une protéine ou un glycane.

La lectine reconnaissant le motif fucose α 1-2 galactose selon la présente invention est avantageusement choisie parmi *Ulex Europaeus Agglutinin I* (UEA-I) et *Trichosanthes Japonica Agglutinin II* (TJA-II). De préférence, il s'agit de *Ulex Europaeus Agglutinin I.*

Afin de garantir que les cellules marquées par la lectine reconnaissant le motif fucose α 1-2 galactose sont des cellules souches cancéreuses colorectales, il est avantageux d'utiliser, en plus de la lectine reconnaissant le motif fucose α 1-2 galactose, un second moyen de marquage des cellules souches cancéreuses colorectales. Ce second moyen de marquage peut être spécifique des cellules souches cancéreuses ou non-cancéreuses.

On peut citer, à titre d'exemple de marqueurs des cellules souches cancéreuses colorectales, CD133, CD44, CD166 (ALCAM), CD24, CD26, CD29, EpCAM, Oct-4 et Sox-2.

De manière préférée, le second moyen de marquage des cellules souches cancéreuses colorectales est un anticorps anti-OCT4 (*octamer-binding transcription factor 4,* codé par le gène POU5F1).

La présente invention concerne donc également l'utilisation d'une lectine reconnaissant le motif fucose α 1-2 galactose, avantageusement choisie parmi *Ulex Europaeus Agglutinin I* (UEA-I) et *Trichosanthes Japonica Agglutinin II* (TJA-II) et d'un anticorps anti-OCT4 pour la détection et/ou la quantification des cellules souches cancéreuses colorectales.

Selon un mode de réalisation, les cellules souches cancéreuses colorectales peuvent être marquées par une lectine reconnaissant le motif fucose α 1-2 galactose, un anticorps anti-OCT4 et au moins une lectine reconnaissant l'antigène T.

La lectine reconnaissant l'antigène T peut être notamment choisie parmi *Agaricus Bisporus Agglutinin* (ABA), *Amaranthus Caudatus Lectin* (ACA) et Jacaline.

On peut également utiliser, pour le marquage des cellules souches cancéreuses colorectales, un mélange de lectines reconnaissant l'antigène T, notamment un mélange de deux lectines choisi parmi ABA et ACA ; ABA et Jacaline ; ACA et Jacaline. Avantageusement, les lectines sont UEA-1, Jacaline et ACA.

Avantageusement, le mélange de trois lectines est constitué d'une lectine reconnaissant le motif fucose α 1-2 galactose : Jacaline : ABA ou ACA, dans un rapport molaire de 625 à 12500 : 16 à 320 : 1 à 20, avantageusement de 5000 à 7000 : 50 à 250 : 5 à 15, notamment de 6250 : 160 : 10.

De manière encore plus avantageuse, la lectine reconnaissant le motif fucose α 1-2 galactose est UEA-1 et les lectines reconnaissant l'antigène T sont Jacaline et ACA dans un rapport molaire UEA-1 : Jacaline : ACA de 6250: 160 : 10.

Afin de détecter et quantifier les cellules souches cancéreuses colorectales, les moyens de marquage des cellules souches cancéreuses colorectales doivent pouvoir être visualisés au sein de l'échantillon biologique.

Les moyens de marquages sont donc liés directement ou indirectement à un moyen de révélation.

Au sens de la présente invention, on entend par « moyen de révélation » une substance ou un ensemble de substances capable de reconnaitre le moyen de marquage et d'émettre un signal détectable au sein du tissu, par exemple par visualisation directe ou spectrophotométrie.

Par « lié directement », on entend au sens de la présente invention que le moyen de révélation est lié de manière covalente au moyen de marquage des cellules cancéreuses colorectales. Le moyen de révélation peut par exemple être un chromophore, un fluorophore ou une enzyme capable de réduire un substrat chromogène, liés de manière covalente au moyen de marquage.

Par « lié indirectement », on entend au sens de la présente invention que le moyen de révélation est lié de manière covalente à une substance secondaire, ladite substance secondaire étant capable de reconnaître de manière spécifique le moyen de marquage. Le moyen de révélation peut par exemple être lié de manière covalente à de l'avidine, de la streptavidine ou à un anticorps anti-biotine et le moyen de marquage être biotinylé.

La présente invention concerne donc également l'utilisation d'une lectine reconnaissant le motif fucose α 1-2 galactose liée à un moyen de révélation A pour la mise en œuvre d'un procédé de détection et/ou quantification des cellules souches cancéreuses colorectales dans un échantillon biologique colorectal.

Le moyen de révélation A peut être un chromophore, un fluorophore, un anticorps reconnaissant le motif fucose α 1-2 galactose lié directement ou indirectement à un fluorophore ou un chromophore, ou une enzyme capable de réduire un substrat chromogène **B.**

Avantageusement, le moyen de révélation **A** est une enzyme capable de réduire un substrat chromogène **B.**

Dans un mode de réalisation, la lectine reconnaissant le motif fucose α 1-2 galactose est biotinylée et le moyen de révélation **A** est lié à de la streptavidine. Avantageusement, le moyen de révélation **A** est une enzyme capable de réduire un substrat chromogène, en particulier une péroxydase de raifort. Ladite enzyme capable de réduire un substrat chromogène **B** peut être intégrée dans un système d'amplification du signal. De tels systèmes d'amplification du signal sont disponibles dans le commerce, par exemple auprès des sociétés Leica ou ThermoFisher Scientific.

Le substrat chromogène **B** dépend de la classe d'enzyme utilisée. Dans le cas de la péroxydase de raifort, il peut par exemple s'agir d'un substrat chromogène choisi dans le groupe constitué de la 3,3',5,5'-tétramethylbenzidine (TMB), de l'*ortho*-phénylènediamine (OPD), de la 3,3'-diaminobenzidine (DAB), de la 10-acetyl-10H-Phenoxazine-3,7-diol (AmplexRed®), de l'acide homovanillique, du luminol, du 3-amino-9-éthylcarbazole (AEC) et de l'acide 2,2'-azino-bis(3-éthylbenzothiazoline-6-sulfonique (ABTS).

Avantageusement, le substrat chromogène **B** est la 3,3'-diaminobenzidine (DAB).

Le second moyen de marquage, de préférence un anticorps anti-OCT4, est lié à un moyen de révélation **C,** avantageusement choisi parmi un chromophore, un fluorophore, ou une enzyme capable de réduire un substrat chromogène **D.**

Ledit moyen de révélation **C** peut être fixé directement ou indirectement au moyen de marquage des cellules souches cancéreuses colorectales.

Avantageusement, le moyen de révélation **C** est lié à un anticorps secondaire reconnaissant le second moyen de marquage. Par exemple, le moyen de marquage peut être un anticorps produit dans une première espèce animale et l'anticorps auquel le moyen de révélation **C** est lié un anticorps produit dans une autre espèce animale reconnaissant un épitope spécifique à l'espèce animale de l'anticorps utilisé comme moyen de marquage, spécifique d'une séquence de chaînes d'immunoglobulines. Alternativement, le moyen de révélation **C** peut être lié à un anticorps tertiaire reconnaissant un anticorps secondaire reconnaissant le second moyen de marquage, notamment un anticorps anti-OCT4.

Dans un mode de réalisation, le second moyen de marquage des cellules souches cancéreuses colorectales est un anticorps anti-OCT4 et le moyen de révélation **C** est lié à un anticorps reconnaissant l'anticorps anti-OCT4. De manière avantageuse, ledit moyen de révélation **C** est une enzyme capable de réduire un substrat chromogène **D,** notamment une phosphatase alcaline. Ladite enzyme capable de réduire un substrat chromogène **D** peut notamment être intégrée à un système d'amplification du signal. De tels systèmes d'amplification du signal sont disponibles dans le commerce, par exemple auprès des sociétés Leica ou Thermofisher Scientific. Dans un mode de réalisation spécifique, l'anticorps anti-OCT4 est un anticorps produit chez le lapin et le moyen de révélation est lié à un anticorps anti-innmunoglobuline de lapin, par exemple de souris.

Le substrat chromogène **D** dépend de la classe d'enzyme utilisée. Dans le cas d'une phosphatase alcaline, il peut par exemple s'agir d'un substrat chromogène choisi dans le groupe constitué du 5-bromo, 4-chloro, 3-indolylphosphate (BCIP), du Nitro-Blue tétrazolium (NBT), d'un mélange BCIP/NBT, du 4-Nitrophenyl phosphate (*p*-NPP), du 3-indoxyl phosphate, du 7-bromo-N-(2-méhoxyphényl)-3-(phosphonooxy)-2-naphthalenecarboxamide et du 3-amino-4-méthoxybenzamide (Fast RED®).

Avantageusement, le substrat chromogène est le 3-amino-4-méthoxybenzamide (Fast RED®) ou un mélange BCIP/NBT.

De manière avantageuse, le substrat chromogène **B** et le substrat chromogène **D** conduisent à la formation de chromophores de couleurs différentes, permettant de différencier les cellules marquées par le premier moyen de marquage et les cellules marquées par le second moyen de marquage.

La présente invention concerne donc également l'utilisation d'une lectine biotinylée reconnaissant le motif fucose α 1-2 galactose et d'un anticorps anti-OCT4, de la streptavidine conjuguée à la péroxydase de raifort et d'un anticorps reconnaissant l'anticorps anti-OCT4 lié à une phosphatase alcaline pour la mise en œuvre d'un procédé de détection et/ou de quantification des cellules souches cancéreuses colorectales. Avantageusement, le substrat chromogène **B** est le DAB et le substrat chromogène **D** est le Fast RED® ou BCIP/NBT.

L'utilisation en tant que moyen de révélation **A** d'une enzyme capable de réduire un substrat chromogène **B** et en tant que moyen de révélation **C** d'une enzyme capable de réduire un substrat chromogène **D** est particulièrement avantageuse dans une méthode immunohistochimique, notamment pour une utilisation sur des coupes histologiques.

Dans un mode de réalisation particulier, la présente invention concerne l'utilisation d'une lectine reconnaissant le motif fucose α 1-2 galactose telle que définie ci-dessus dans une méthode de détection immunohistochimique.

Dans ce mode de réalisation particulier, l'échantillon biologique est une coupe histologique d'un échantillon biologique colorectal, notamment d'une résection tumorale ou d'une biopsie colorectale.

Dans ce mode de réalisation particulier, le moyen de révélation **A** peut être lié de manière covalente à la lectine reconnaissant le motif fucose α 1-2 galactose. Le moyen de révélation **A** peut également être lié à de l'avidine, de la streptavidine ou à un anticorps anti-biotine et la lectine reconnaissant le motif fucose α 1-2 galactose est biotinylée.

Dans ce mode de réalisation particulier, le moyen de révélation **C** peut être lié de manière covalente au second moyen de marquage ou lié de manière covalente à un anticorps secondaire reconnaissant le second moyen de marquage. Le moyen de révélation **C** peut également être lié de manière covalente à un anticorps tertiaire reconnaissant un anticorps secondaire reconnaissant second moyen de marquage. De préférence, le second moyen de marquage est un anticorps anti-OCT4 et le moyen de révélation **C** est lié de manière covalente à l'anticorps anti-OCT4 ou lié de manière covalente à un anticorps secondaire reconnaissant l'anticorps anti-OCT4. Le moyen de révélation **C** peut également être lié de manière covalente à un anticorps tertiaire reconnaissant un anticorps secondaire reconnaissant l'anticorps anti-OCT4.

Dans ce mode de réalisation particulier, la présente invention concerne de manière préférée l'utilisation d'une lectine biotinylée reconnaissant le motif fucose α 1-2 galactose, d'une enzyme capable de réduire un substrat chromogène **B** liée à de la streptavidine, d'un anticorps anti-OCT4, d'une enzyme capable de réduire un substrat chromogène **D** lié à un anticorps secondaire reconnaissant l'anticorps anti-OCT4, pour la détection et/ou la quantification des cellules souches cancéreuses colorectales dans une coupe histologique d'un tissu colorectal.

De manière préférée entre toutes, la lectine biotinylée reconnaissant le motif fucose α 1-2 galactose est UEA-1, le second moyen de marquage est un anticorps anti-OCT4, le moyen de révélation **A** est une péroxydase de raifort, le substrat chromogène **B** est la diaminobenzidine, le moyen de révélation **C** est une phosphatase alcaline liée à un anticorps secondaire, le substrat chromogène **D** est le 3-amino-4-méthoxybenzamide (Fast RED®).

Un second objet de la présente invention concerne un procédé de détection et/ou de quantification *in vitro* des cellules souches cancéreuses colorectales dans un échantillon biologique colorectal, comprenant une étape de révélation d'une lectine reconnaissant le motif fucose α 1-2 galactose dans un échantillon biologique colorectal marquées par ladite lectine.

Par «étape de révélation », on entend au sens de la présente invention l'étape visant à détecter visuellement, par des méthodes spectroscopiques ou spectrophotométriques, la lectine reconnaissant le motif fucose α 1-2 galactose liée aux cellules souches cancéreuses colorectales, après la mise en contact de ladite lectine avec l'échantillon biologique.

La présente invention concerne plus particulièrement un procédé de détection et/ou de quantification *in vitro* tel que défini ci-dessus, comprenant les étapes de :
(a) marquage des cellules souches cancéreuses colorectales avec une lectine reconnaissant le motif fucose α 1-2 galactose dans ledit échantillon biologique, pour obtenir des cellules souches cancéreuses colorectales marquées dans ledit échantillon biologique,
(b) révélation de ladite lectine dans l'échantillon biologique colorectal,
(c) détection et/ou quantification des cellules souches cancéreuses colorectales marquées par ladite lectine dans ledit tissu biologique.

L'étape de marquage est précédée d'étapes classiques destinées à préparer l'échantillon biologique, telles que le lavage du tissu, l'utilisation d'un tampon destiné à démasquer les antigènes (dans le cas du double marquage, le pH est avantageusement de 6).

L'étape de marquage est avantageusement réalisée dans un diluant ayant un pouvoir saturant et capable d'empêcher les fixations aspécifiques (par exemple un diluant PBS-BSA).

L'étape de marquage est mise en œuvre à une température comprise entre 10 et 30 °C, avantageusement pendant un temps d'incubation allant de 10 à 30 minutes.

La présente invention concerne donc avantageusement un procédé de détection et/ou de quantification des cellules souches cancéreuses colorectales dans une coupe histologique de tissu colorectal, comprenant les étapes de :
(a2) marquage des cellules souches cancéreuses colorectales de la coupe histologique avec une lectine biotinylée reconnaissant le motif fucose α 1-2 galactose, pour obtenir des cellules souches cancéreuses colorectales marquées dans ladite coupe histologique,
(b2) mise en contact de la coupe histologique obtenue à l'étape (a2) avec un moyen de révélation A lié à de la streptavidine, de l'avidine ou un anticorps anti-biotine,
(c2) révélation de la lectine reconnaissant le motif fucose α 1-2 galactose,
(d2) détection et/ou quantification des cellules souches cancéreuses colorectales marquées par ladite lectine dans la coupe histologique.

De manière avantageuse, le procédé décrit ci-dessus peut être mis en œuvre à l'étape (a) avec une lectine reconnaissant le motif fucose α 1-2 galactose et au moins une lectine reconnaissant l'antigène T, avantageusement choisie parmi ACA, ABA et Jacaline, plus avantageusement avec un mélange de lectines reconnaissant l'antigène T choisi parmi un mélange ABA et ACA, un mélange ABA et Jacaline et un mélange ACA et Jacaline, avantageusement ACA et Jacaline.

Avantageusement, le mélange de lectines est constitué d'une lectine reconnaissant le motif fucose α 1-2 galactose : Jacaline : ABA ou ACA, dans un rapport molaire de 625 à 12500 : 16 à 320 : 1 à 20, avantageusement de 5000 à 7000 : 50 à 250 : 5 à 15, notamment de 6250 : 160 : 10.

De manière particulièrement avantageuse, l'étape (a) est mise en œuvre avec un mélange UEA-1, ACA et Jacaline, de préférence dans un ratio UEA-1 : Jacaline : ACA de 6250: 160 : 10.

Le procédé décrit ci-dessus peut être précédé par une étape de marquage avec un second moyen de marquage des cellules souches cancéreuses tel que défini ci-dessus. En particulier, le procédé décrit ci-dessus peut être précédé d'une étape de marquage par un anticorps anti-OCT4.

La présente invention concerne donc un procédé comprenant préalablement à l'étape (a) ou (a2) de marquage par la lectine reconnaissant le motif fucose α 1-2 galactose les étapes de :
(a'1) marquage des cellules souches cancéreuses colorectales dudit échantillon biologique colorectal avec un second moyen de marquage, de préférence un anticorps anti-OCT4, pour obtenir des cellules souches cancéreuses colorectales marquées par un anticorps anti-OCT4 dans ledit échantillon biologique,
(a'2) révélation dudit second moyen de marquage, de préférence l'anticorps anti-OCT4.

L'étape de marquage est précédée d'étapes classiques destinées à préparer l'échantillon biologique, telles que le lavage du tissu, l'utilisation d'un tampon destiné à démasquer les antigènes (dans le cas du double marquage, le pH est avantageusement de 6).

L'étape de marquage est avantageusement réalisée dans un diluant ayant un pouvoir saturant et capable d'empêcher les accroches aspécifiques (par exemple un diluant PBS-BSA).

L'étape de marquage est mise en œuvre à une température comprise entre 10 et 30 °C, avantageusement pendant un temps d'incubation allant de 10 à 30 minutes.

La présente invention concerne donc également un procédé tel que décrit ci-dessus, comprenant les étapes de :
(a3) marquage des cellules souches cancéreuses colorectales avec un second moyen de marquage des cellules souches cancéreuses colorectales, de préférence un anticorps anti-OCT4, dans une coupe histologique d'un tissu colorectal, pour obtenir des cellules souches cancéreuses marquées par ledit second moyen de marquage dans ladite coupe histologique,
(b3) mise en contact de la coupe histologique obtenue à l'étape (a3) avec un anticorps secondaire reconnaissant le second moyen de marquage, de préférence l'anticorps anti-OCT4, lié à un moyen de révélation C,
(c3) révélation du second moyen de marquage,
(d3) marquage des cellules souches cancéreuses colorectales de la coupe histologique obtenue à l'étape (c3) avec une lectine biotinylée reconnaissant le motif fucose α 1-2 galactose, et éventuellement au moins une lectine biotinylée reconnaissant l'antigène T, pour obtenir des cellules souches cancéreuses marquées par la lectine reconnaissant le motif fucose α 1-2 galactose et éventuellement marquées par la au moins une lectine reconnaissant l'antigène T dans ladite coupe histologique,
(e3) mise en contact de la coupe histologique obtenue à l'étape (d3) avec un moyen de révélation A lié à de la streptavidine, de l'avidine ou un anticorps anti-biotine,
(f3) révélation de la lectine reconnaissant le motif fucose α 1-2 galactose et de l'éventuellement au moins une lectine reconnaissant l'antigène T,
(g3) détection et/ou quantification des cellules souches cancéreuses colorectales marquées par la lectine reconnaissant le motif fucose α 1-2 galactose et le second moyen de marquage, de préférence l'anticorps anti-OCT4 dans la coupe histologique.

Dans un mode de réalisation particulier, la présente invention concerne un procédé de détection et/ou de quantification des cellules souches cancéreuses colorectales dans une coupe histologique colorectale, comprenant les étapes de :
(a4) marquage des cellules souches cancéreuses colorectales avec un anticorps anti-OCT4 dans une coupe histologique d'un tissu colorectal, pour obtenir des cellules souches colorectales marquées par un anticorps anti-OCT4 dans ladite coupe histologique,
(b4) mise en contact de la coupe histologique obtenue à l'étape (a4) avec un anticorps secondaire reconnaissant l'anticorps anti-OCT4 lié à une enzyme capable de réduire un substrat chromogène,
(c4) révélation de l'anticorps anti-OCT4 avec un substrat chromogène,
(d4) marquage des cellules souches cancéreuses colorectales de la coupe histologique obtenue à l'étape (c4) avec une lectine biotinylée reconnaissant le motif fucose α 1-2 galactose, et éventuellement au moins une lectine biotinylée reconnaissant l'antigène T, pour obtenir des cellules souches cancéreuses colorectales marquées par la lectine reconnaissant le motif fucose α 1-2 galactose et éventuellement la au moins une lectine reconnaissant l'antigène T dans ladite coupe histologique,
(e4) mise en contact de la coupe histologique obtenue à l'étape (d4) avec de la streptavidine, de l'avidine ou un anticorps anti-biotine lié à une enzyme capable de réduire un substrat chromogène,
(f4) révélation de la lectine reconnaissant le motif fucose α 1-2 galactose et de l'éventuellement au moins une lectine reconnaissant l'antigène T avec un substrat chromogène,
(g4) détection et/ou quantification des cellules souches cancéreuses colorectales marquées par la lectine reconnaissant le motif fucose α 1-2 galactose, éventuellement la au moins une lectine biotinylée reconnaissant l'antigène T, et l'anticorps anti-OCT4 dans la coupe histologique.

Dans un mode de réalisation préféré, la présente invention concerne un procédé de détection et/ou de quantification des cellules souches cancéreuses colorectales dans une coupe histologique colorectale, comprenant les étapes de :
(a5) marquage des cellules souches cancéreuses colorectales avec un anticorps anti-OCT4 dans une coupe histologique d'un tissu colorectal, pour obtenir des cellules souches colorectales marquées par un anticorps anti-OCT4 dans ladite coupe histologique,
(b5) mise en contact de la coupe histologique obtenue à l'étape (a5) avec un anticorps secondaire reconnaissant l'anticorps anti-OCT4 lié à une phosphatase alcaline,
(c5) révélation de l'anticorps anti-OCT4 avec du FastRED® ou un mélange BCIP/NBT, (d5) marquage des cellules souches cancéreuses colorectales de la coupe histologique obtenue à l'étape (c5) avec UEA-1 biotinylée, et éventuellement ACA biotinylée et Jacaline biotinylée dans un ratio UEA-1 : Jacaline : ACA de 6250 : 160 : 10, pour obtenir des cellules souches cancéreuses colorectales marquées par UEA-1 et éventuellement ACA et Jacaline,
(e5) mise en contact de la coupe histologique obtenue à l'étape (d5) avec de la streptavidine liée à la péroxydase de raifort,
(f5) révélation d'UEA-1 et éventuellement ACA et Jacaline avec DAB,
(g5) détection et/ou quantification des cellules souches cancéreuses colorectales marquées par UEA-1 et l'anticorps anti-OCT4 et éventuellement ACA et Jacaline dans la coupe histologique.

Dans un autre mode de réalisation, la méthodologie générale décrite dans la présente demande peut être mise en œuvre en utilisant, pour la détection et/ou la quantification des cellules souches cancéreuses colorectales, la cytométrie en flux. Dans un mode de réalisation, la détection et/ou la quantification des cellules souches cancéreuses peut être réalisée par cytométrie en flux. Cette méthode permet également l'isolement des cellules souches cancéreuses colorectales en utilisant un équipement approprié.

L'échantillon biologique peut être une biopsie d'un tissu tumoral colorectal dans laquelle les cellules ont été préalablement dissociées les unes des autres, par exemple avec le produit Liberase™, commercialisé par la société Roche Diagnostic, qui permet la dissociation du tissu sans affecter les glycanes de surface.

Afin de détecter et/ou quantifier les cellules par cytométrie en flux, la lectine reconnaissant le motif fucose α 1-2 galactose est liée à un fluorophore E directement ou indirectement. Le second moyen de marquage, de préférence un anticorps anti-OCT4, est lié à un fluorophore F directement ou indirectement, par exemple à un anticorps secondaire reconnaissant l'anticorps anti-OCT4. Avantageusement, les fluorophores **E** et **F** sont différents l'un de l'autre. De préférence, le fluorophore **E** est Alexa 488 lié à de la streptavidine et la lectine reconnaissant le motif fucose α 1-2 galactose est biotinylée et le fluorophore **F** est Alexa 633 lié directement à l'anticorps anti-OCT4.

La présente invention concerne donc également un procédé de détection et/ou de quantification des cellules souches cancéreuses colorectales tel que décrit ci-dessus, dans un échantillon biologique colorectal, dans lequel l'étape de détection est mise en œuvre par cytométrie en flux, avec des fluorophores comme moyen de révélation.

La méthode selon la présente invention permettant la détection et la quantification des cellules souches cancéreuses colorectales, elle est particulièrement adaptée au diagnostic du cancer colorectal, en particulier par l'intermédiaire d'un examen anatomopathologique ou histopathologique.

Les cellules souches cancéreuses se différencient des autres cellules composant les tumeurs. Elles sont capables de s'auto-renouveler et sont résistantes aux traitements conventionnels de chimiothérapie et de radiothérapie. Les cellules souches cancéreuses seraient donc à l'origine de la récidive tumorale après une chimiothérapie/radiothérapie conventionnelle pourtant capable d'éliminer les tumeurs. La détection de ces cellules dans un tissu tumoral présente donc un intérêt fondamental pour évaluer le risque de récidive chez un patient traité par chimiothérapie ou radiothérapie. La quantification des cellules souches cancéreuses est également cruciale pour déterminer l'agressivité d'un cancer. En outre, la détection des cellules souches cancéreuses dans un tissu tumoral permet d'établir un pronostic pendant le traitement, ouvrant ainsi la possibilité de modifier le traitement pour cibler de manière plus efficace ces cellules, et ainsi réduire l'agressivité de la tumeur et les risques de récidive.

La présente invention concerne donc l'utilisation d'une lectine reconnaissant le motif fucose α 1-2 galactose, et éventuellement d'un anticorps anti-OCT4, pour le diagnostic *in vitro* du risque de récidive d'un cancer colorectal et/ou de l'agressivité d'un cancer colorectal pour définir une valeur pronostique pour l'adaptation thérapeutique d'un cancer colorectal.

Dans un mode de réalisation, le marquage est mis en œuvre avec une lectine reconnaissant le motif fucose α 1-2 galactose, avantageusement UEA-1, un anticorps anti-OCT4 et au moins une lectine reconnaissant l'antigène T, avantageusement choisie parmi ACA, ABA et Jacaline ou un mélange de lectines reconnaissant l'antigène T choisi parmi un mélange ABA et ACA, un mélange ABA et Jacaline et un mélange ACA et Jacaline, avantageusement ACA et Jacaline. De manière avantageuse, le ratio UEA-1 : Jacaline : ACA est de 6250: 160 : 10.

La présente invention concerne plus particulièrement une méthode de diagnostic *in vitro* du risque de récidive et/ou de l'agressivité et/ou de la valeur pronostique d'un traitement du cancer colorectal comprenant les étapes de :
(a) marquage des cellules souches cancéreuses colorectales dans un échantillon biologique colorectal avec une lectine reconnaissant le motif fucose α 1-2 galactose, pour obtenir des cellules souches cancéreuses colorectales marquées par la lectine reconnaissant le motif fucose α 1-2 galactose dans ledit échantillon biologique,
(b) révélation des cellules souches cancéreuses colorectales dans le tissu colorectal,
(c) détection et/ou quantification des cellules souches cancéreuses marquées par la lectine reconnaissant le motif fucose α 1-2 galactose,
(d) déduction du risque de récidive et/ou de l'agressivité et/ou de la valeur pronostique du traitement du cancer colorectal à partir de la quantité de cellules souches cancéreuses colorectales.

Avantageusement, la présente invention est mise en œuvre avec une lectine reconnaissant le motif fucose α 1-2 galactose et un second moyen de marquage des cellules cancéreuses colorectales, de préférence d'un anticorps anti-OCT4.

Les Inventeurs ont en effet mis en évidence qu'un double marquage conduisait à une sensibilité et une spécificité optimisées réduisant ainsi drastiquement le risque de faux positifs qui pourrait résulter du marquage de cellules non-souches cancéreuses ou de cellules souches non-cancéreuses par la lectine reconnaissant le motif fucose α 1-2 galactose.

La présente invention concerne donc également une méthode de diagnostic *in vitro* du risque de récidive et/ou de l'agressivité pour définir une valeur pronostique pour l'adaptation thérapeutique d'un cancer colorectal comprenant les étapes de :
(a6) marquage des cellules souches cancéreuses colorectales avec un second moyen de marquage, de préférence un anticorps anti-OCT4 dans une coupe histologique d'un tissu colorectal, pour obtenir des cellules souches cancéreuses colorectales marquées dans ladite coupe histologique,
(b6) mise en contact de la coupe histologique obtenue à l'étape (a6) avec un anticorps secondaire reconnaissant le second moyen de marquage lié à un moyen de révélation C, (c6) révélation du second moyen de marquage, de préférence l'anticorps anti-OCT4,
(d6) marquage des cellules souches cancéreuses colorectales de la coupe histologique obtenue à l'étape (c6) avec une lectine biotinylée reconnaissant le motif fucose α 1-2 galactose et éventuellement au moins une lectine biotinylée reconnaissant l'antigène T, pour obtenir des cellules souches cancéreuses colorectales marquées par la lectine biotinylée reconnaissant le motif fucose α 1-2 galactose et la éventuellement au moins une lectine biotinylée reconnaissant l'antigène T dans la coupe histologique,
(e6) mise en contact de la coupe histologique obtenue à l'étape (d6) avec de la streptavidine, de l'avidine ou un anticorps anti-biotine lié à un moyen de révélation A, (f6) révélation de la lectine reconnaissant le motif fucose α 1-2 galactose et de l'éventuellement au moins une lectine reconnaissant l'antigène T,
(g6) détection et/ou quantification des cellules souches cancéreuses colorectales dans la coupe histologique obtenue à l'étape (f6),
(h6) déduction du risque de récidive et/ou de l'agressivité et/ou de la valeur pronostique du traitement du cancer colorectal à partir de la présence et/ou du nombre de cellules souches cancéreuses colorectales.

Dans un mode de réalisation préféré, la présente invention concerne une méthode de diagnostic *in vitro* du risque de récidive et/ou de l'agressivité et/ou de la valeur pronostique d'un traitement du cancer colorectal comprenant les étapes de :
(a7) marquage des cellules souches cancéreuses colorectales avec un anticorps anti-OCT4 dans une coupe histologique d'un tissu colorectal, pour obtenir des cellules souches cancéreuses colorectales marquées par l'anticorps anti-OCT4 dans ladite coupe histologique,
(b7) mise en contact de la coupe histologique obtenue à l'étape (a7) avec un anticorps secondaire reconnaissant l'anticorps anti-OCT4 lié à un moyen de révélation C, de préférence une enzyme capable de réduire un substrat chromogène, notamment une phosphatase alcaline,
(c7) révélation de l'anticorps anti-OCT4, de préférence avec du Fast Red® ou un mélange BCIP/NBT,
(d7) marquage des cellules souches cancéreuses colorectales de la coupe histologique obtenue à l'étape (c7) avec UEA-1 biotinylée ou un mélange UEA-1 : Jacaline : ACA dans un rapport molaire de 6250 : 160 : 10, pour obtenir des cellules souches cancéreuses colorectales marquées par la lectine biotinylée reconnaissant le motif fucose α 1-2 galactose et la éventuellement au moins une lectine biotinylée reconnaissant l'antigène T dans la coupe histologique,
(e7) mise en contact de la coupe histologique obtenue à l'étape (d7) avec de la streptavidine, de l'avidine ou un anticorps anti-biotine lié à un moyen de révélation A, de préférence une enzyme capable de réduire un substrat chromogène,
(f7) révélation d'UEA-1 ou UEA-1, Jacaline et ACA, de préférence avec de la DAB, (g7) détection et/ou quantification des cellules souches cancéreuses colorectales dans la coupe histologique obtenue à l'étape (f7),
(h7) déduction du risque de récidive et/ou de l'agressivité et/ou de la valeur pronostique du traitement du cancer colorectal à partir de la présence et/ou du nombre de cellules souches cancéreuses colorectales.

Un cinquième objet de la présente invention concerne un kit, notamment destiné à la détection et/ou la quantification des cellules souches cancéreuses colorectales, comprenant :
- une lectine biotinylée reconnaissant le motif fucose α 1-2 galactose et éventuellement au moins une lectine biotinylée reconnaissant l'antigène T,
- de la streptavidine liée à une péroxydase de raifort, avantageusement intégrée dans un système d'amplification du signal,
- un anticorps anti-OCT4,
- un anticorps secondaire reconnaissant l'anticorps anti-OCT4 lié à une phosphatase alcaline capable de réduire un substrat chromogène, avantageusement intégrée dans un système d'amplification.

Le kit selon la présente invention peut également, outre les parties décrites ci-dessus contenir un substrat chromogène susceptible d'être réduit par la péroxydase de raifort et un substrat chromogène susceptible d'être réduit par la phosphatase alcaline.

Le kit selon la présente invention peut également contenir des solutions tampons, des solutions de lavage, des diluants et un manuel d'utilisation.

Dans un mode de réalisation préféré, la présente invention concerne un kit, notamment destiné à la détection et/ou la quantification des cellules souches cancéreuses colorectales, comprenant :
- UEA-1 biotinylée et éventuellement ACA biotinylée et Jacaline biotinylée,
- un anticorps anti-OCT4,
- un anticorps secondaire reconnaissant l'anticorps anti-OCT4, lié à une phosphatase alcaline,
- de la streptavidine liée à une péroxydase de raifort,
- éventuellement de la DAB,
- éventuellement du Fast Red® ou un mélange BCIP/NBT,
- éventuellement des solutions de lavage,
- éventuellement des diluants,
- éventuellement des solutions tampon.

La présente invention concerne également l'utilisation d'un kit tel que défini ci-dessus pour la mise en œuvre d'un procédé *in vitro* de détection et/ou de quantification des cellules souches cancéreuses colorectales dans une coupe histologique de tissu colorectal.

La présente invention concerne en outre l'utilisation d'un kit tel que défini ci-dessus pour la mise en œuvre d'une méthode de diagnostic *in vitro* du risque de récidive et/ou de l'agressivité d'un cancer colorectal pour définir une valeur pronostique pour l'adaptation thérapeutique d'un cancer colorectal.

Dans un septième objet, la présente invention concerne une méthode de détection et/ou de quantification *in vitro* des cellules souches cancéreuses colorectales dans un échantillon biologique colorectal, comprenant une étape de révélation d'une lectine reconnaissant le motif fucose α 1-2 galactose modifiée avec un moyen de révélation dans un échantillon biologique colorectal marquées par ladite lectine.

La présente invention concerne plus particulièrement une méthode de détection et/ou de quantification *in vitro* telle que définie ci-dessus, comprenant les étapes de :
(a) marquage des cellules souches cancéreuses colorectales avec une lectine reconnaissant le motif fucose α 1-2 galactose modifiée avec un moyen de révélation dans ledit échantillon biologique, pour obtenir des cellules souches cancéreuses colorectales marquées dans ledit échantillon biologique,
(b) révélation de ladite lectine dans l'échantillon biologique colorectal,
(c) détection et/ou quantification des cellules souches cancéreuses colorectales marquées par ladite lectine dans ledit tissu biologique.

La présente invention concerne donc avantageusement une méthode de détection et/ou de quantification des cellules souches cancéreuses colorectales dans une coupe histologique de tissu colorectal telle que définie ci-dessus, dans laquelle le moyen de révélation est la biotine, comprenant les étapes de :
(a2) marquage des cellules souches cancéreuses colorectales de la coupe histologique avec une lectine biotinylée reconnaissant le motif fucose α 1-2 galactose, pour obtenir des cellules souches cancéreuses colorectales marquées dans ladite coupe histologique,
(b2) mise en contact de la coupe histologique obtenue à l'étape (a2) avec un moyen de révélation A lié à de la streptavidine, de l'avidine ou un anticorps anti-biotine,
(c2) révélation de la lectine reconnaissant le motif fucose α 1-2 galactose,
(d2) détection et/ou quantification des cellules souches cancéreuses colorectales marquées par ladite lectine dans la coupe histologique.

De manière avantageuse, le procédé décrit ci-dessus peut être mis en œuvre à l'étape (a) avec une lectine reconnaissant le motif fucose α 1-2 galactose modifiée avec un moyen de révélation et au moins une lectine reconnaissant l'antigène T modifiée avec un moyen de révélation avantageusement choisie parmi ACA, ABA et Jacaline, plus avantageusement avec un mélange de lectines reconnaissant l'antigène T modifiées avec un moyen de révélation choisi parmi un mélange ABA et ACA, un mélange ABA et Jacaline et un mélange ACA et Jacaline, avantageusement ACA et Jacaline.

Avantageusement, le mélange de lectines est constitué d'une lectine reconnaissant le motif fucose α 1-2 galactose : Jacaline modifiée avec un moyen de révélation : ABA modifiée avec un moyen de révélation ou ACA modifiée avec un moyen de révélation, dans un rapport molaire de 625 à 12500 : 16 à 320 : 1 à 20, avantageusement de 5000 à 7000 : 50 à 250 : 5 à 15, notamment de 6250 : 160 : 10.

De manière particulièrement avantageuse, l'étape (a) est mise en œuvre avec un mélange UEA-1 modifiée avec un moyen de révélation, ACA modifiée avec un moyen de révélation et Jacaline modifiée avec un moyen de révélation, de préférence dans un ratio UEA-1 : Jacaline : ACA de 6250: 160 : 10.

Le procédé décrit ci-dessus peut être précédé par une étape de marquage avec un second moyen de marquage des cellules souches cancéreuses tel que défini ci-dessus. En particulier, le procédé décrit ci-dessus peut être précédé d'une étape de marquage par un anticorps anti-OCT4.

La présente invention concerne donc un procédé comprenant préalablement à l'étape (a) ou (a2) de marquage par la lectine reconnaissant le motif fucose α 1-2 galactose les étapes de :
(a'1) marquage des cellules souches cancéreuses colorectales dudit échantillon biologique colorectal avec un second moyen de marquage, de préférence un anticorps anti-OCT4, pour obtenir des cellules souches cancéreuses colorectales marquées par un anticorps anti-OCT4 dans ledit échantillon biologique,
(a'2) révélation dudit second moyen de marquage, de préférence l'anticorps anti-OCT4.

La présente invention concerne donc également une méthode telle que décrite ci-dessus, comprenant les étapes de :
(a3) marquage des cellules souches cancéreuses colorectales avec un second moyen de marquage des cellules souches cancéreuses colorectales, de préférence un anticorps anti-OCT4, dans une coupe histologique d'un tissu colorectal, pour obtenir des cellules souches cancéreuses marquées par ledit second moyen de marquage dans ladite coupe histologique,
(b3) mise en contact de la coupe histologique obtenue à l'étape (a3) avec un anticorps secondaire reconnaissant le second moyen de marquage, de préférence l'anticorps anti-OCT4, lié à un moyen de révélation C,
(c3) révélation du second moyen de marquage,
(d3) marquage des cellules souches cancéreuses colorectales de la coupe histologique obtenue à l'étape (c3) avec une lectine biotinylée reconnaissant le motif fucose α 1-2 galactose, et éventuellement au moins une lectine biotinylée reconnaissant l'antigène T, pour obtenir des cellules souches cancéreuses marquées par la lectine reconnaissant le motif fucose α 1-2 galactose et éventuellement marquées par la au moins une lectine reconnaissant l'antigène T dans ladite coupe histologique,
(e3) mise en contact de la coupe histologique obtenue à l'étape (d3) avec un moyen de révélation A lié à de la streptavidine, de l'avidine ou un anticorps anti-biotine,
(f3) révélation de la lectine reconnaissant le motif fucose α 1-2 galactose et de l'éventuellement au moins une lectine reconnaissant l'antigène T,
(g3) détection et/ou quantification des cellules souches cancéreuses colorectales marquées par la lectine reconnaissant le motif fucose α 1-2 galactose et le second moyen de marquage, de préférence l'anticorps anti-OCT4 dans la coupe histologique.

Dans un mode de réalisation particulier, la présente invention concerne une méthode de détection et/ou de quantification des cellules souches cancéreuses colorectales dans une coupe histologique colorectale, comprenant les étapes de :
(a4) marquage des cellules souches cancéreuses colorectales avec un anticorps anti-OCT4 dans une coupe histologique d'un tissu colorectal, pour obtenir des cellules souches colorectales marquées par un anticorps anti-OCT4 dans ladite coupe histologique,
(b4) mise en contact de la coupe histologique obtenue à l'étape (a4) avec un anticorps secondaire reconnaissant l'anticorps anti-OCT4 lié à une enzyme capable de réduire un substrat chromogène,
(c4) révélation de l'anticorps anti-OCT4 avec un substrat chromogène,
(d4) marquage des cellules souches cancéreuses colorectales de la coupe histologique obtenue à l'étape (c4) avec une lectine biotinylée reconnaissant le motif fucose α 1-2 galactose, et éventuellement au moins une lectine biotinylée reconnaissant l'antigène T, pour obtenir des cellules souches cancéreuses colorectales marquées par la lectine reconnaissant le motif fucose α 1-2 galactose et éventuellement la au moins une lectine reconnaissant l'antigène T dans ladite coupe histologique,
(e4) mise en contact de la coupe histologique obtenue à l'étape (d4) avec de la streptavidine, de l'avidine ou un anticorps anti-biotine lié à une enzyme capable de réduire un substrat chromogène,
(f4) révélation de la lectine reconnaissant le motif fucose α 1-2 galactose et de l'éventuellement au moins une lectine reconnaissant l'antigène T avec un substrat chromogène,
(g4) détection et/ou quantification des cellules souches cancéreuses colorectales marquées par la lectine reconnaissant le motif fucose α 1-2 galactose, éventuellement la au moins une lectine biotinylée reconnaissant l'antigène T, et l'anticorps anti-OCT4 dans la coupe histologique.

Dans un mode de réalisation préféré, la présente invention concerne une méthode de détection et/ou de quantification des cellules souches cancéreuses colorectales dans une coupe histologique colorectale, comprenant les étapes de :
(a5) marquage des cellules souches cancéreuses colorectales avec un anticorps anti-OCT4 dans une coupe histologique d'un tissu colorectal, pour obtenir des cellules souches colorectales marquées par un anticorps anti-OCT4 dans ladite coupe histologique,
(b5) mise en contact de la coupe histologique obtenue à l'étape (a5) avec un anticorps secondaire reconnaissant l'anticorps anti-OCT4 lié à une phosphatase alcaline,
(c5) révélation de l'anticorps anti-OCT4 avec du FastRED® ou un mélange BCIP/NBT,
(d5) marquage des cellules souches cancéreuses colorectales de la coupe histologique obtenue à l'étape (c5) avec UEA-1 biotinylée, et éventuellement ACA biotinylée et Jacaline biotinylée dans un ratio UEA-1 : Jacaline : ACA de 6250 : 160 : 10, pour obtenir des cellules souches cancéreuses colorectales marquées par UEA-1 et éventuellement ACA et Jacaline,
(e5) mise en contact de la coupe histologique obtenue à l'étape (d5) avec de la streptavidine liée à la péroxydase de raifort,
(f5) révélation d'UEA-1 et éventuellement ACA et Jacaline avec DAB,
(g5) détection et/ou quantification des cellules souches cancéreuses colorectales marquées par UEA-1 et l'anticorps anti-OCT4 et éventuellement ACA et Jacaline dans la coupe histologique.

Dans un autre mode de réalisation, la méthodologie générale décrite dans la présente demande peut être mise en œuvre en utilisant, pour la détection et/ou la quantification des cellules souches cancéreuses colorectales, la cytométrie en flux. Dans un mode de réalisation, la détection et/ou la quantification des cellules souches cancéreuses peut être réalisée par cytométrie en flux. Cette méthode permet également l'isolement des cellules souches cancéreuses colorectales en utilisant un équipement approprié.

L'échantillon biologique peut être une biopsie d'un tissu tumoral colorectal dans laquelle les cellules ont été préalablement dissociées les unes des autres, par exemple avec le produit Liberase™, commercialisé par la société Roche Diagnostic, qui permet la dissociation du tissu sans affecter les glycanes de surface.

Afin de détecter et/ou quantifier les cellules par cytométrie en flux, la lectine reconnaissant le motif fucose α 1-2 galactose est liée à un fluorophore E directement ou indirectement. Le second moyen de marquage, de préférence un anticorps anti-OCT4, est lié à un fluorophore F directement ou indirectement, par exemple à un anticorps secondaire reconnaissant l'anticorps anti-OCT4. Avantageusement, les fluorophores E et F sont différents l'un de l'autre. De préférence, le fluorophore E est Alexa 488 lié à de la streptavidine et la lectine reconnaissant le motif fucose α 1-2 galactose est biotinylée et le fluorophore F est Alexa 633 lié directement à l'anticorps anti-OCT4.

La présente invention concerne donc également une méthode de détection et/ou de quantification des cellules souches cancéreuses colorectales telle que décrite ci-dessus, dans un échantillon biologique colorectal, dans lequel l'étape de détection est mise en œuvre par cytométrie en flux, avec des fluorophores comme moyen de révélation.

Dans un mode de réalisation, le marquage est mis en œuvre avec une lectine reconnaissant le motif fucose α 1-2 galactose modifiée avec un moyen de révélation, avantageusement UEA-1, un anticorps anti-OCT4 et au moins une lectine reconnaissant l'antigène T modifiée avec un moyen de révélation, avantageusement choisie parmi ACA, ABA et Jacaline ou un mélange de lectines reconnaissant l'antigène T modifiée avec un moyen de révélation choisi parmi un mélange ABA et ACA, un mélange ABA et Jacaline et un mélange ACA et Jacaline, avantageusement ACA et Jacaline. De manière avantageuse, le ratio UEA-1 : Jacaline : ACA est de 6250: 160 : 10.

La présente invention concerne plus particulièrement une méthode de diagnostic *in vitro* du risque de récidive et/ou de l'agressivité et/ou de la valeur pronostique d'un traitement du cancer colorectal comprenant les étapes de :
(a) marquage des cellules souches cancéreuses colorectales dans un échantillon biologique colorectal avec une lectine reconnaissant le motif fucose α 1-2 galactose modifiée avec un moyen de révélation, pour obtenir des cellules souches cancéreuses colorectales marquées par la lectine reconnaissant le motif fucose α 1-2 galactose dans ledit échantillon biologique,
(b) révélation des cellules souches cancéreuses colorectales dans le tissu colorectal,
(c) détection et/ou quantification des cellules souches cancéreuses marquées par la lectine reconnaissant le motif fucose α 1-2 galactose,
(d) déduction du risque de récidive et/ou de l'agressivité et/ou de la valeur pronostique du traitement du cancer colorectal à partir de la quantité de cellules souches cancéreuses colorectales.

Avantageusement, la présente invention est mise en œuvre avec une lectine reconnaissant le motif fucose α 1-2 galactose modifiée avec un moyen de révélation et un second moyen de marquage des cellules cancéreuses colorectales, de préférence d'un anticorps anti-OCT4.

Les Inventeurs ont en effet mis en évidence qu'un double marquage conduisait à une sensibilité et une spécificité optimisées réduisant ainsi drastiquement le risque de faux positifs qui pourrait résulter du marquage de cellules non-souches cancéreuses ou de cellules souches non-cancéreuses par la lectine reconnaissant le motif fucose α 1-2 galactose.

La présente invention concerne donc également une méthode de diagnostic *in vitro* du risque de récidive et/ou de l'agressivité pour définir une valeur pronostique pour l'adaptation thérapeutique d'un cancer colorectal comprenant les étapes de :
(a6) marquage des cellules souches cancéreuses colorectales avec un second moyen de marquage, de préférence un anticorps anti-OCT4 dans une coupe histologique d'un tissu colorectal, pour obtenir des cellules souches cancéreuses colorectales marquées dans ladite coupe histologique,
(b6) mise en contact de la coupe histologique obtenue à l'étape (a6) avec un anticorps secondaire reconnaissant le second moyen de marquage lié à un moyen de révélation C, (c6) révélation du second moyen de marquage, de préférence l'anticorps anti-OCT4,
(d6) marquage des cellules souches cancéreuses colorectales de la coupe histologique obtenue à l'étape (c6) avec une lectine biotinylée reconnaissant le motif fucose α 1-2 galactose et éventuellement au moins une lectine biotinylée reconnaissant l'antigène T, pour obtenir des cellules souches cancéreuses colorectales marquées par la lectine biotinylée reconnaissant le motif fucose α 1-2 galactose et la éventuellement au moins une lectine biotinylée reconnaissant l'antigène T dans la coupe histologique,
(e6) mise en contact de la coupe histologique obtenue à l'étape (d6) avec de la streptavidine, de l'avidine ou un anticorps anti-biotine lié à un moyen de révélation A, (f6) révélation de la lectine reconnaissant le motif fucose α 1-2 galactose et de l'éventuellement au moins une lectine reconnaissant l'antigène T,
(g6) détection et/ou quantification des cellules souches cancéreuses colorectales dans la coupe histologique obtenue à l'étape (f6),
(h6) déduction du risque de récidive et/ou de l'agressivité et/ou de la valeur pronostique du traitement du cancer colorectal à partir de la présence et/ou du nombre de cellules souches cancéreuses colorectales.

Dans un mode de réalisation préféré, la présente invention concerne une méthode de diagnostic *in vitro* du risque de récidive et/ou de l'agressivité et/ou de la valeur pronostique d'un traitement du cancer colorectal comprenant les étapes de :
(a7) marquage des cellules souches cancéreuses colorectales avec un anticorps anti-OCT4 dans une coupe histologique d'un tissu colorectal, pour obtenir des cellules souches cancéreuses colorectales marquées par l'anticorps anti-OCT4 dans ladite coupe histologique,
(b7) mise en contact de la coupe histologique obtenue à l'étape (a7) avec un anticorps secondaire reconnaissant l'anticorps anti-OCT4 lié à un moyen de révélation C, de préférence une enzyme capable de réduire un substrat chromogène, notamment une phosphatase alcaline,
(c7) révélation de l'anticorps anti-OCT4, de préférence avec du Fast Red® ou un mélange BCIP/NBT,
(d7) marquage des cellules souches cancéreuses colorectales de la coupe histologique obtenue à l'étape (c7) avec UEA-1 biotinylée ou un mélange UEA-1 : Jacaline : ACA dans un rapport molaire de 6250 : 160 : 10, pour obtenir des cellules souches cancéreuses colorectales marquées par la lectine biotinylée reconnaissant le motif fucose α 1-2 galactose et la éventuellement au moins une lectine biotinylée reconnaissant l'antigène T dans la coupe histologique,
(e7) mise en contact de la coupe histologique obtenue à l'étape (d7) avec de la streptavidine, de l'avidine ou un anticorps anti-biotine lié à un moyen de révélation A, de préférence une enzyme capable de réduire un substrat chromogène,
(f7) révélation d'UEA-1 ou UEA-1, Jacaline et ACA, de préférence avec de la DAB,
(g7) détection et/ou quantification des cellules souches cancéreuses colorectales dans la coupe histologique obtenue à l'étape (f7),
(h7) déduction du risque de récidive et/ou de l'agressivité et/ou de la valeur pronostique du traitement du cancer colorectal à partir de la présence et/ou du nombre de cellules souches cancéreuses colorectales.

### DESCRIPTION DES FIGURES

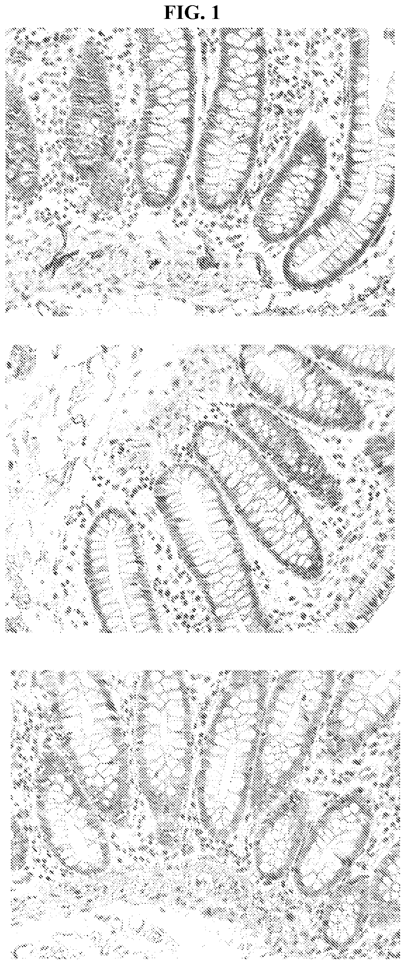

La **Figure 1** représente des coupes histologiques sériées d'un tissu sain marquées par UEA1 (figure du haut), un anticorps anti-OCT4 (figure médiane) et doublement marquées par UEA-1 / anticorps anti-OCT4 (figure du bas). On note dans le tissu sain l'absence de coloration due au double marquage.

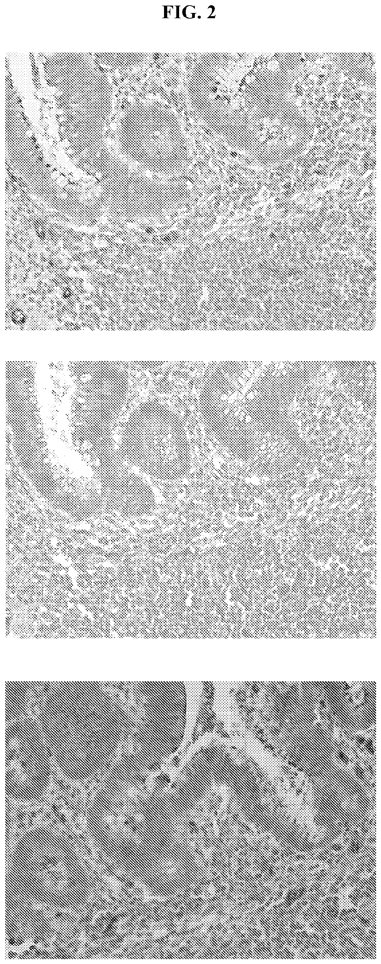

La **Figure 2** représente des coupes histologiques sériées d'un tissu tumoral marquées par UEA1 (figure du haut), un anticorps anti-OCT4 (figure médiane) et doublement marquées par UEA-1 / anticorps anti-OCT4 (figure du bas). On observe dans ce tissu tumoral une importante coloration sombre (de couleur marron sur l'image obtenue en pratique) résultant du double marquage par UEA-1 et l'anticorps anti-OCT4.

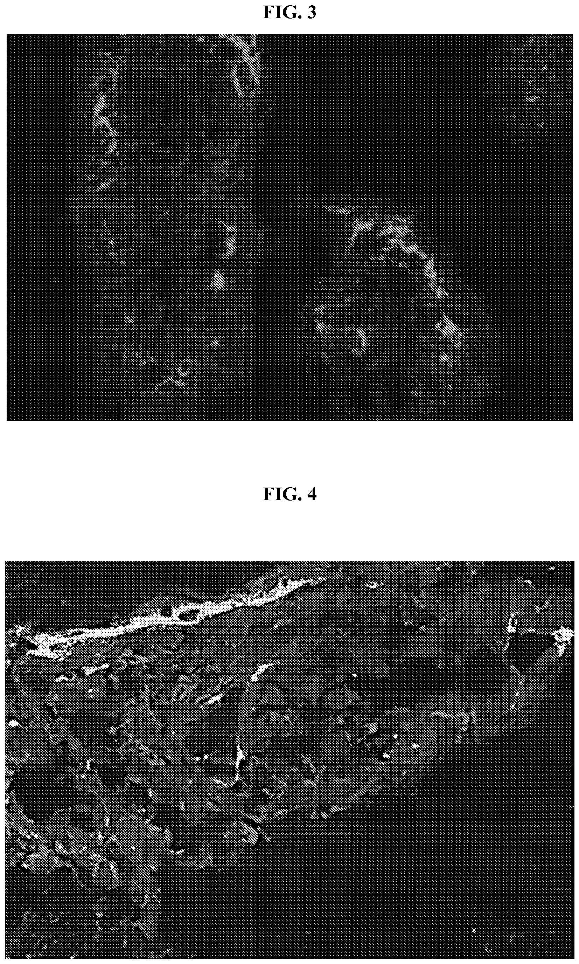

La **Figure 3** montre l'image d'un tissu sain doublement marqué par UEA-1 / streptavidine conjuguée avec Alexa Fluor 488 et un anticorps anti-OCT4 conjugué avec Alexa Fluor 594. L'absence de fluorescence indique l'absence de cellules souches cancéreuses colorectales.
La **Figure 4** montre l'image d'un tissu tumoral doublement marqué par UEA-1 / streptavidine conjuguée avec Alexa Fluor 488 et un anticorps anti-OCT4 conjugué avec Alexa Fluor 594. Les zones claires sur l'image sont dues au double marquage des cellules souches cancéreuses par UEA-1 / streptavidine conjuguée avec Alexa Fluor 488 et l'anticorps anti-OCT4 conjugué avec Alexa Fluor 594. Le double marquage permet de mettre en évidence la présence d'un nombre important de cellules souches cancéreuses colorectales.

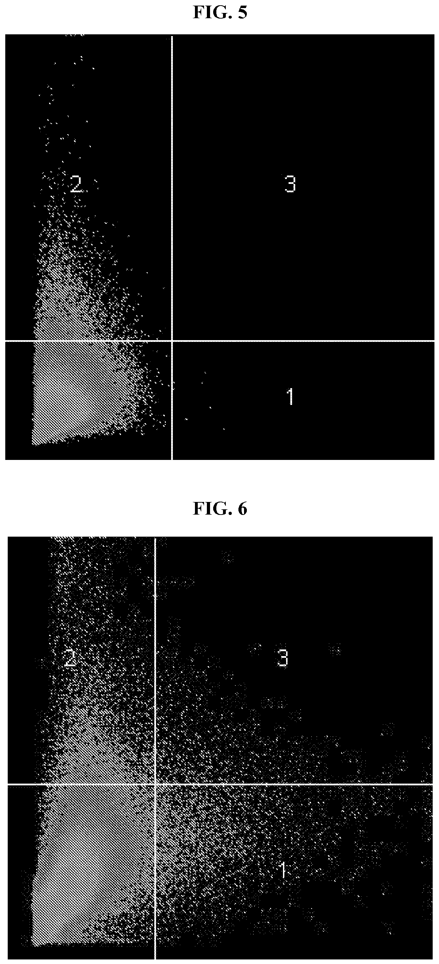

La **Figure 5** montre l'image retraitée de microscopie confocale après utilisation de la méthode en z-stack obtenue par double marquage d'un tissu sain avec UEA-1 / streptavidine conjuguée avec Alexa Fluor 488 et un anticorps anti-OCT4 conjugué avec Alexa Fluor 594. Le quadrant supérieur gauche correspond aux cellules marquées par l'anticorps anti-OCT4, le quadrant inférieur gauche correspond aux cellules non marquées et le quadrant inférieur droit correspond aux cellules marquées par UEA-1. Le quadrant supérieur droit correspond aux cellules doublement marquées par UEA-1 et l'anticorps anti-OCT4. L'absence de cellules doublement marquées confirme que le tissu ne contient pas de cellules souches colorectales.
La **Figure 6** montre l'image retraitée de microscopie confocale après utilisation de la méthode en z-stack obtenue par double marquage d'un tissu tumoral avec UEA-1 / streptavidine conjuguée avec Alexa Fluor 488 et un anticorps anti-OCT4 conjugué avec Alexa Fluor 594. Le quadrant supérieur gauche correspond aux cellules marquées par l'anticorps anti-OCT4, le quadrant inférieur gauche correspond aux cellules non marquées et le quadrant inférieur droit correspond aux cellules marquées par UEA-1. Le quadrant supérieur droit correspond aux cellules doublement marquées par UEA-1 et l'anticorps anti-OCT4. La présence de cellules dans le quadrant supérieur droit indique la présence de 16,6 % de cellules souches cancéreuses colorectales.

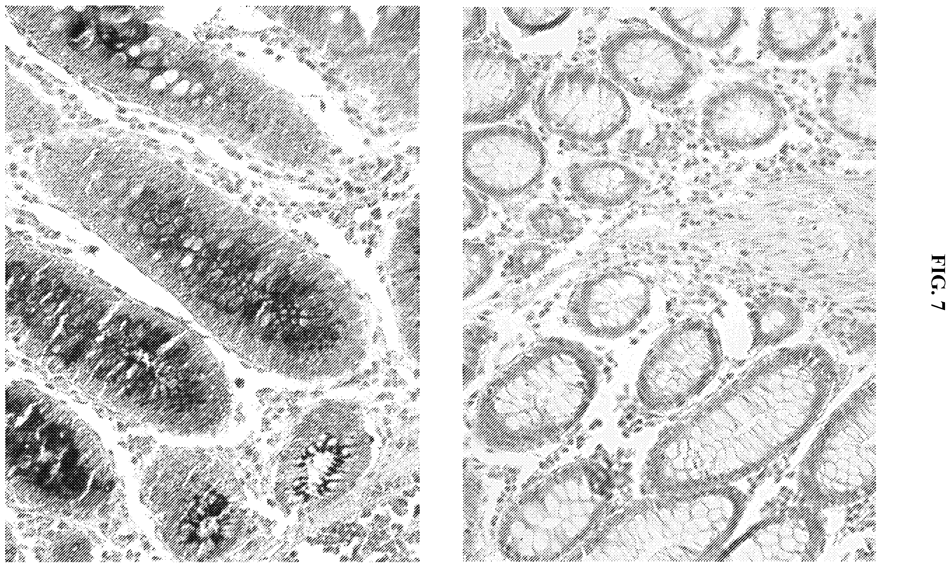

La **Figure 7** montre l'image d'un tissu sain doublement marqué par l'anticorps anti-OCT4 et UEA-1 (image du haut) et l'image d'un tissu tumoral doublement marqué par l'anticorps anti-OCT4 et UEA-1 dans lequel est présent un nombre important de cellules souches cancéreuses colorectales (image du bas). Le double marquage est absent du tissu sain alors que de nombreuses cellules sont marquées dans le tissu tumoral (taches sombres, de couleur marron sur l'image obtenue en pratique).

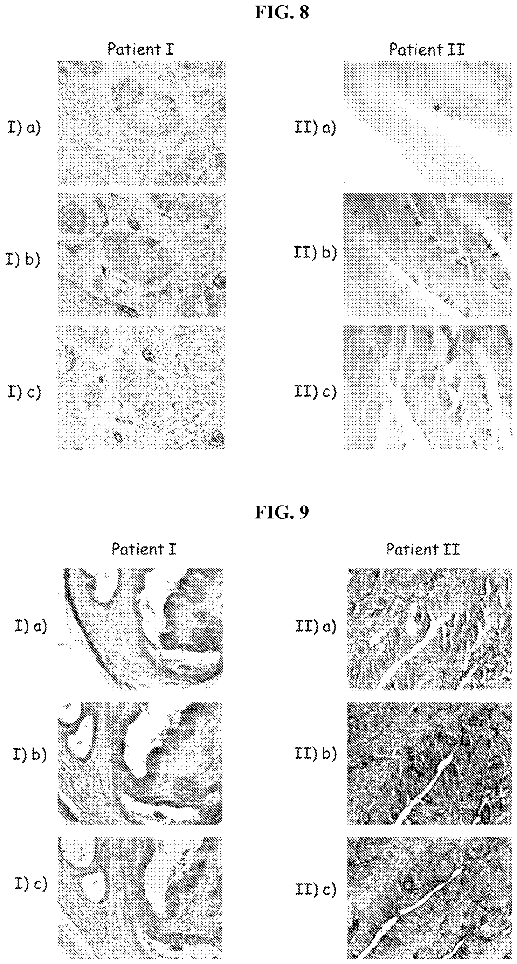

Les **Figures 8 à 11** montrent des résultats de marquage histochimique sur les coupes de tissus tumoraux de patients atteints de cancers colorectaux à différents stades de progression, correspondant à l'étude clinique rétrospective décrite à l'exemple 3.
La **Figure 8** montre les résultats obtenus pour un patient atteint d'un cancer colorectal de stade I ayant survécu (cas favorable, patient I) et d'un patient atteint d'un cancer colorectal de stade I n'ayant pas survécu (cas défavorable, patient II). Le marquage est réalisé soit avec l'anticorps anti-Oct4 (la et IIa), soit avec l'anticorps anti-Oct4 et un mélange de lectines UEA-1/ACA/Jacaline (Ib et IIb), ou avec un mélange de lectines UEA-1/ACA/Jacaline (Ic et IIc).
La **Figure 9** montre les résultats obtenus pour un patient atteint d'un cancer colorectal de stade II ayant survécu (cas favorable, patient I) et d'un patient atteint d'un cancer colorectal de stade II n'ayant pas survécu (cas défavorable, patient II). Le marquage est réalisé soit avec l'anticorps anti-Oct4 (la et IIa), soit avec l'anticorps anti-Oct4 et UEA-1 (Ib et IIb) ou avec UEA-1 (Ic et IIc).

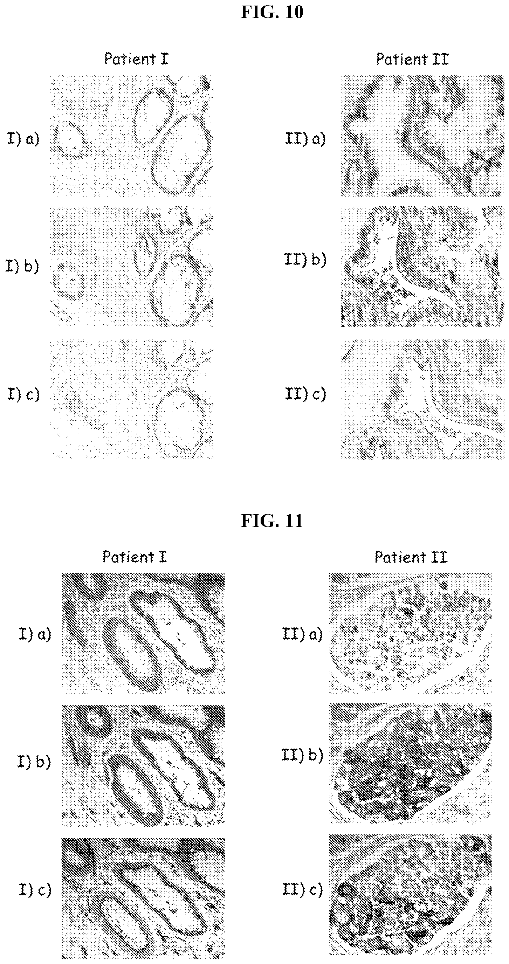

La **Figure 10** montre les résultats obtenus pour un patient atteint d'un cancer colorectal de stade III ayant survécu (cas favorable, patient I) et d'un patient atteint d'un cancer colorectal de stade III n'ayant pas survécu (cas défavorable, patient II). Le marquage est réalisé soit avec l'anticorps anti-Oct4 (la et IIa), soit avec l'anticorps anti-Oct4 et un mélange de lectines UEA-1/ACA/Jacaline (Ib et IIb) ou avec un mélange de lectines UEA-1/ACA/Jacaline (Ic et IIc).
La **Figure 11** montre les résultats obtenus pour un patient atteint d'un cancer colorectal de stade IV ayant survécu avec un traitement de chimiothérapie (cas favorable, patient I) et d'un patient atteint d'un cancer colorectal de stade IV n'ayant pas survécu (cas défavorable, patient II). Le marquage est réalisé soit avec l'anticorps anti-Oct4 (la et IIa), soit avec l'anticorps anti-Oct4 et UEA-1 (Ib et IIb) ou avec UEA-1 (Ic et IIc).

### EXEMPLES :

### Exemple 1 : double marquage visible sur coupe histologique

### Protocole de double marquage Lectine(s) / OCT4 en paraffine :

Matériel utilisé : Blocs de paraffine, Glace, Microtome, Lames superfrost®, Automate Bond Max (Leica Microsystems) avec ordinateur, Consommables Leica (alcool, tampon de lavage, tampon ER1, tampon dewax, étiquettes, coverslips, tubes), tampon PBS-BSA 5%, Anticorps anti-Oct-4 (ThermoFisher Scientific), Kit Bond polymer Refine red détection (Leica), Lectines biotinylées (UEA-1, Jacalin et ACA) (Vector Lab), Kit Bond Intense R détection (Leica), milieu de montage Leica, lamelles et microscope.

Les blocs de paraffine contenant les prélèvements de CCR provenant de chacun des patients identifiés par leur numéro (donné par le service d'anatomie pathologique) ont été placés dans la glace pendant environ 1 heure afin d'être refroidis ceci afin de faciliter leur coupe au microtome à une épaisseur de 4µm.

Des lames dites « superfrost », ceci pour un maximum d'adhésion du tissu coupé ont été identifiées par les mêmes numéros que ceux présents sur les blocs. Une goutte d'eau a été placée au centre de chacune de ces lames.

Les coupes ont été réalisées au microtome et placées sur la goutte d'eau préalablement déposée. Les lames ont par la suite été posées sur une plaque chauffante à 37°C afin de faciliter leur adhésion et l'excédent d'eau a été retiré. L'ensemble des lames réalisé a été placé au sein d'une étuve à 37°C dans l'objectif de les sécher.

La suite de la manipulation a fait intervenir l'automate Bond Max de chez Leica relié à un ordinateur possédant un logiciel pilotant l'automate.

Durant le temps où les lames sont à l'étuve, l'ensemble de la manipulation de double marquage a été préparé en commençant par la vérification du niveau sur l'automate de chacun des produits nécessaires à la réalisation de la manipulation, puis à l'identification des lames avec leur même numéro ceci sur le logiciel pilotant l'automate. Des étiquettes permettant un protocole standardisé ont été générées. La dilution des anticorps et leur quantité ont été calculées et les différents kits nécessaires préparés. Il est à noter que chacun des produits utilisés a dû être scanné et le niveau remis à zéro avant chacune des expérimentations effectuées.

Les étiquettes ont par la suite été collées sur leurs lames correspondantes à la sortie de l'étuve et des *coverslips* ont été placés sur chacune d'entre elles pour permettre au produit d'être présent sur toute la surface de la lame grâce aux propriétés de contact.

Le portoir de lames a été placé dans l'automate et après reconnaissance par le lecteur de chacun des éléments et des lames identifiés par leurs codes-barres présents sur les étiquettes, la manipulation a été initiée.

Elle a commencé par un déparaffinage à la chaleur grâce au produit Dewax de chez Leica permettant par la suite l'accessibilité des anticorps. Cette étape ainsi que toutes les autres a été suivie de lavages, grâce au Bond Wash 10X préalablement dilué, ceci à trois reprises.

Cette étape a été suivie d'un prétraitement pendant 5 min avec le tampon ER1 de chez Leica qui présente un pH=6 permettant de démasquer les antigènes à atteindre dans le cadre de ce double marquage.

L'anticorps anti-OCT4 (lapin, ThermoFisher Scientific) a été placé en premier au 1/500^{ème} pendant 20 min, la dilution s'effectuant en PBS-BSA 5% afin d'empêcher les fixations aspécifiques de par son pouvoir saturant.

Le kit Bond polymer Refine red détection de chez Leica (anticorps anti-lapin conjugué à adaptateur phosphatase alcaline polymérique) a permis par la suite de révéler cet anticorps grâce au Fast Red® dans le visible en couleur rouge.

La lectine biotinylée ou un mélange de lectines biotinylées soit UEA-1 au 1/40^{ème} ou le mélange de lectines au 1/3 composé de UEA-1 au 1/40^{ème} avec la Jacaline au 1/400^{ème} et ACA au 1/25000^{ème} ceci toujours à l'aide du diluant PBS BSA-5% a été placé pendant 20 min. Il est à noter que l'ordre des marquages est important pour permettre le bon fonctionnement de la manipulation.

Le kit Bond Intense R detection, grâce à l'intervention d'une streptavidine-HRP jouant le rôle d'anticorps secondaire a permis de par ses propriétés de révéler cette ou ces lectines biotinylées en marron grâce aux propriétés de la DAB venant reconnaitre le complexe biotine/streptavidine-HRP.

Une étape de contre coloration bleutée grâce à la présence d'hématoxyline a ensuite été réalisée pendant 7 min afin de rendre l'ensemble du prélèvement identifiable.

Les lames ont été retirées de l'automate. Les coupes ont été ensuite réhydratées en plongeant les lames manuellement dans un bain d'alcool par deux fois pendant 5 min. Cette étape de réhydratation a été poursuivie par un bain de toluène durant 5 min également.

Les lames ont pu dès lors être montées en mettant une goutte de milieu de montage (Leica). Ce milieu particulier est une colle biologique permettant l'absence d'altération et de dissolution des marquages respectifs ceci surtout pour celui présentant une coloration rouge.

Les lames ont enfin été observées au microscope et des clichés ont été pris au grossissement 20.

Les résultats sont présentés dans les figures 1 et 2.

Le double marquage d'un tissu sain par UEA-1 et par un anticorps anti-OCT4 a montré l'absence de double marquage des cellules. Il peut donc en être déduit l'absence de cellules souches cancéreuses colorectales dans ce tissu.

Dans un tissu tumoral coexistent plusieurs types cellulaires : des cellules non-souches tumorales, des cellules souches non-tumorales, des cellules souches tumorales et des cellules non-souches non-tumorales. Le marquage d'un tissu tumoral par UEA-1 seul ou UEA-1 et un anticorps anti-OCT4 met en évidence la capacité de la lectine UEA-1 à marquer sélectivement les cellules souches cancéreuses colorectales (zones les plus sombres). OCT-4 est capable de marquer les cellules souches (tumorales et non tumorales) et permet de confirmer que les cellules marquées par UEA-1 sont des cellules souches cancéreuses colorectales.

### Exemple 2 : double marquage fluorescent sur coupe histologique :

Des coupes de 4 à 6 µm ont été incubées :
- en présence d'anticorps anti-OCT4 (lapin, dilution 1/200^{ème}) pendant une nuit à 4 °C, puis les coupes ont été lavées trois fois avec PBS et incubées 1 heure avec un anticorps secondaire anti-lapin conjugué à Alexa Fluor 594 (ThermoFisher Scientific).
- en présence d'UEA-1 biotinylée (dilution 1/200^{ème}) pendant 20 à 30 min à température ambiante, puis les coupes ont été lavées trois fois avec PBS et révélées par de la streptavidine 488 conjuguée à Alexa Fluor 488 pendant 30 min.
- en présence d'anticorps anti-OCT4 (lapin, dilution 1/200^{ème}) pendant une nuit à 4 °C, puis les coupes ont été lavées trois fois avec PBS et incubées 1 heure avec un anticorps secondaire anti-lapin conjugué à Alexa Fluor 594 (ThermoFisher Scientific). Les coupes sont ensuite incubées en présence d'UEA-1 biotinylée (dilution 1/200^{ème}) pendant 20 à 30 min à température ambiante, lavées trois fois avec PBS et révélées par de la streptavidine conjuguée à Alexa Fluor 488 pendant 30 min.

Après trois lavages dans le PBS, les noyaux ont été colorés avec DAPI (1/10000^{ème}) pendant 5 minutes. Après 3 lavages supplémentaires avec DAPI dans l'eau, les coupes ont été montées sur des lames et observées sur un microscope confocal LSM 510 META (Zeiss) en utilisant le logiciel associé.

Les résultats sont présentés dans les figures 3 et 4.

Dans un tissu sain (Figure 3), un double marquage n'a pas été observé. Dans un tissu tumoral, au contraire, un double marquage des cellules souches cancéreuses colorectales peut être mis en évidence (Figure 4, zone claire de l'image).

Les cellules incubées avec un anticorps anti-OCT4 conjugué à Alexa Fluor 594 et UEA-1 biotinylée / streptavidine conjuguée à Alexa Fluor 488 ont été analysées par microscopie confocale et l'image retraitée par la méthode en z-stack.

Les résultats sont présentés dans les figures 5 et 6.

Dans le tissu sain (Figure 5), aucune cellule n'est doublement marquée, indiquant l'absence de cellules souches cancéreuses colorectales.

Dans le tissu tumoral, l'analyse de l'image retraitée permet de détecter des cellules doublement marquées par UEA-1 et l'anticorps anti-OCT4. La quantité de ces cellules représente 16,6 % du nombre total de cellules.

A partir de ces données, il est possible de conclure que la tumeur contient des cellules souches cancéreuses colorectales susceptible de conduire à une récidive. L'agressivité de la tumeur étant avérée, ainsi que le risque de récidive, il peut donc être conclu que le traitement doit être poursuivi et adapté en conséquence.

### Exemple 3 :

Une étude clinique rétrospective sur des tissus tumoraux de 100 patients atteints de cancers colorectaux à différents stades de progression a été réalisée.

Cette étude a pour but de valider le test de l'agressivité du cancer colorectal par une mesure immunohistochimique grâce à des lectines spécifiques des cellules souches cancéreuses. Les prélèvements ont été volontairement choisis dans une cohorte de patients datant de 5 ans de manière à pouvoir connaitre la survie des patients de l'étude sur 60 mois. Le suivi sur 5 ans du devenir des patients permet aussi de passer au-delà du délai d'action des traitements par chimiothérapie qui est estimé à 3 ans.

Pour chaque stade de cancer deux exemples sont présentés, le cas favorable d'un prélèvement négatif pour les marqueurs de cellules souches cancéreuses et pour lequel le patient a survécu après 60 mois. Le cas défavorable d'un prélèvement positif pour les marqueurs ces cellules souches cancéreuses et pour lequel le patient n'a pas survécu.

Pour les exemples de cancer colorectal de stade I, les patients ont subi une ablation de la tumeur et n'ont pas reçu de chimiothérapie. Il s'agit là d'un protocole classique de traitement du cancer colorectal de stade I.

Pour les exemples de cancer colorectal de stade II, les patients ont subi une ablation de la tumeur et n'ont pas reçu de chimiothérapie. Il s'agit là d'un protocole classique de traitement du cancer colorectal de stade II présentant un nombre suffisant de ganglions périphériques.

Pour les exemples de cancer colorectal de stade III, les patients ont subi une ablation de la tumeur et n'ont pas reçu de chimiothérapie. Il s'agit là de cas spécifiques de traitement du cancer colorectal de stade III, généralement les patients subissent aussi une chimiothérapie en complément de la chirurgie.

Pour les exemples de cancer colorectal de stade IV, les patients ont subi une ablation de la tumeur et reçoivent systématiquement un traitement par chimiothérapie. Dans l'exemple défavorable présenté ici le patient est décédé avant de recevoir le traitement par chimiothérapie.

Le marquage a été réalisé de la manière suivante :

### Cancer colorectal de stade 1 :

Double marquage : OCT4 + Mélange de lectines (UEA-1, Jacalin, ACA)
Marqueur glycosylé : Mix de lectines (UEA-1, Jacalin, ACA)

### Cancer colorectal de stade II :

Double marquage : OCT4 + lectine UEA-1 seule
Marqueur glycosylé : lectine UEA-1 seule

### Cancer colorectal de stade III :

Double marquage : OCT4 + Mix de lectines (UEA-1, Jacalin, ACA)
Marqueur glycosylé : Mix de lectines (UEA-1, Jacalin, ACA)

### Cancer colorectal de stade IV :

Double marquage : OCT4 + lectine UEA-1 seule
Marqueur glycosylé : lectine UEA-1 seule

Pour chaque stade (I, II et III) l'analyse permet d'identifier des groupes de patients positifs et négatifs au test (présentant ou non des cellules souches cancéreuses). Le test permet d'apporter un complément d'information pour le praticien et d'envisager un traitement ou suivi particulier pour les patients positifs.

Les résultats sont présentés dans les Figures 8 à 11.

## Revendications

1. Utilisation d'une lectine reconnaissant le motif fucose α 1-2 galactose, avantageusement UEA-1 ou TJA-II, en tant que premier moyen de marquage des cellules souches cancéreuses colorectales, pour la détection et/ou la quantification desdites cellules souches cancéreuses colorectales.

2. Utilisation selon la revendication 1, d'une lectine reconnaissant le motif fucose α 1-2 galactose et d'au moins un second moyen de marquage des cellules souches cancéreuses colorectales, de préférence un anticorps anti-OCT4.

3. Utilisation selon la revendication 2, d'une lectine reconnaissant le motif fucose α 1-2 galactose, d'un anticorps anti-OCT4 et d'au moins une lectine reconnaissant l'antigène T, avantageusement choisie parmi ACA, ABA et Jacaline.

4. Utilisation selon la revendication 3, dans laquelle un moyen de révélation A de la lectine reconnaissant le motif fucose α 1-2 galactose, avantageusement choisi parmi un chromophore, un fluorophore, et une enzyme capable de réduire un substrat chromogène B est conjugué à de la streptavidine, ladite lectine reconnaissant le motif fucose α 1-2 galactose étant biotinylée.

5. Utilisation selon l'une des revendications 2 à 4, dans laquelle un moyen de révélation C de l'anticorps anti-OCT4, avantageusement choisi parmi un chromophore, un fluorophore, ou une enzyme capable de réduire un substrat chromogène D est lié à un anticorps secondaire reconnaissant l'anticorps anti-OCT4.

6. Utilisation selon l'une des revendications 1 à 5, dans laquelle l'échantillon biologique colorectal est une coupe histologique.

7. Utilisation selon l'une des revendications 1 à 6, dans laquelle :
• l'échantillon biologique est une coupe histologique,
• la lectine reconnaissant le motif fucose α 1-2 galactose est UEA-1 biotinylée,
• le moyen de révélation **A** est une péroxydase de raifort,
• le substrat chromogène **B** est la diaminobenzidine,
• le moyen de révélation **C** est une phosphatase alcaline liée à un anticorps secondaire,
• le substrat chromogène **D** est le 3-amino-4-méthoxybenzamide.

8. Procédé de détection et/ou de quantification *in vitro* des cellules souches cancéreuses colorectales dans un échantillon biologique colorectal, comprenant une étape de révélation d'une lectine reconnaissant le motif fucose α 1-2 galactose dans un échantillon biologique colorectal marqué par ladite lectine.

9. Procédé de détection et/ou de quantification *in vitro* selon la revendication 8, comprenant les étapes de :
(a) **marquage** des cellules souches cancéreuses colorectales avec une lectine reconnaissant le motif fucose α 1-2 galactose dans ledit échantillon biologique, pour obtenir des cellules souches cancéreuses colorectales marquées dans ledit échantillon biologique,
(b) **révélation** de ladite lectine dans l'échantillon biologique colorectal,
(c) détection et/ou quantification des cellules souches cancéreuses colorectales marquées par ladite lectine dans ledit tissu biologique.

10. Utilisation d'une lectine reconnaissant le motif fucose α 1-2 galactose, et éventuellement d'un anticorps anti-OCT4, pour le diagnostic *in vitro* du risque de récidive d'un cancer colorectal et/ou de l'agressivité d'un cancer colorectal pour définir la valeur pronostique pour l'adaptation thérapeutique d'un cancer colorectal.

11. Méthode de diagnostic *in vitro* du risque de récidive et/ou de l'agressivité d'un cancer colorectal pour définir une valeur pronostique pour l'adaptation thérapeutique d'un cancer colorectal comprenant les étapes de :
(a3) **marquage** des cellules souches cancéreuses colorectales avec un anticorps anti-OCT4 dans une coupe histologique d'un tissu colorectal, pour obtenir des cellules souches cancéreuses colorectales dans ladite coupe histologique,
(b3) mise en contact de la coupe histologique obtenue à l'étape (a3) avec un anticorps secondaire reconnaissant l'anticorps anti-OCT4 lié à un moyen de révélation C,
(c3) **révélation** de l'anticorps anti-OCT4,
(d3) **marquage** des cellules souches cancéreuses colorectales de la coupe histologique obtenue à l'étape (c3) avec une lectine biotinylée reconnaissant le motif fucose α 1-2 galactose et éventuellement au moins une lectine biotinylée reconnaissant l'antigène T, pour obtenir des cellules souches cancéreuses colorectales marquées par la lectine biotinylée reconnaissant le motif fucose α 1-2 galactose et la éventuellement au moins une lectine biotinylée reconnaissant l'antigène T dans la coupe histologique,
(e3) mise en contact de la coupe histologique obtenue à l'étape (d3) avec de la streptavidine, de l'avidine ou un anticorps anti-biotine lié à un moyen de révélation **A,**
(f3) **révélation** de la lectine reconnaissant le motif fucose α 1-2 galactose et de l'éventuellement au moins une lectine reconnaissant l'antigène T,
(g3) détection et/ou quantification des cellules souches cancéreuses colorectales dans la coupe histologique obtenue à l'étape (f3),
(h3) déduction du risque de récidive et/ou de l'agressivité d'un cancer colorectal pour définir une valeur pronostique pour l'adaptation thérapeutique d'un cancer colorectal à partir de la présence et/ou le nombre de cellules souches cancéreuses colorectales.

12. Kit comprenant :
• une lectine biotinylée reconnaissant le motif fucose α 1-2 galactose et éventuellement au moins une lectine biotinylée reconnaissant l'antigène T, avantageusement choisie parmi ABA, ACA et Jacaline,
• de la streptavidine liée à une péroxydase de raifort,
• un anticorps anti-OCT4,
• un anticorps secondaire reconnaissant l'anticorps anti-OCT4 lié à une phosphatase alcaline.

## Patentansprüche

1. Verwendung eines Lektins, das die Fucose-α 1-2 Galactose-Einheit erkennt, vorteilhafterweise UEA-1 oder TJA-II, als erstes Markierungsmittel der kolorektalen Krebsstammzellen zur Erfassung und/oder Quantifizierung der kolorektalen Krebsstammzellen.

2. Verwendung nach Anspruch 1 eines Lektins, das die Fucose-a 1-2 Galactose-Einheit erkennt, und mindestens eines zweiten Markierungsmittels der kolorektalen Krebsstammzellen, vorzugsweise eines Anti-OCT4-Antikörpers.

3. Verwendung nach Anspruch 2 eines Lektins, das die Fucose-a 1-2 Galactose-Einheit erkennt, eines Anit-OCT4-Antikörpers und mindestens eines Lektins, das das T-Antigen erkennt, vorteilhafterweise ausgewählt unter ACA, ABA und Jacalin.

4. Verwendung nach Anspruch 3, bei der ein Mittel zum Sichtbarmachen A des Lektins, das die Fucose-a 1-2 Galactose-Einheit erkennt, vorteilhafterweise ausgewählt unter einem Chromophor, einem Fluorophor, und ein Enzym, das geeignet ist, ein chromogenes Substrat B zu reduzieren, mit Streptavidin konjugiert ist, wobei das Lektin, das die Fucose-a 1-2 Galactose-Einheit erkennt, biotinyliert ist.

5. Verwendung nach einem der Ansprüche 2 bis 4, bei der ein Mittel zur Sichtbarmachung C des Anti-OCT4-Antikörpers, vorteilhafterweise ausgewählt unter einem Chromophor, einem Fluorophor, oder ein Enzym, das geeignet ist, ein chromogenes Substrat D zu reduzieren, an einen sekundären Antikörper, der den Anti-OCT4-Antikörper erkennt, gebunden ist.

6. Verwendung nach einem der Ansprüche 1 bis 5, bei der die kolorektale biologische Probe ein histologischer Schnitt ist.

7. Verwendung nach einem der Ansprüche 1 bis 6, bei der:
• die biologische Probe ein histologischer Schnitt ist,
• das Lektin, das die Fucose-a 1-2 Galactose-Einheit erkennt, biotinyliertes UEA-1 ist,
• das Mittel zur Sichtbarmachung A eine Meerrettichperoxidase ist,
• das chromogene Substrat B Diaminobenzidin ist,
• das Mittel zur Sichtbarmachung C eine Alkali-Phosphatase ist, die an einen sekundären Antikörper gebunden ist,
• das chromogene Substrat D 3-Amino-4-Methoxybenzamid ist.

8. Verfahren zur In-Vitro-Erfassung und/oder Quantifizierung der kolorektalen Krebsstammzellen in einer kolorektalen biologischen Probe, umfassend einen Schritt der Schichtbarmachung eines Lektins, das die Fucose-a 1-2 Galactose-Einheit erkennt, in einer kolorektalen biologischen Probe, die durch das Lektin markiert ist.

9. Verfahren zur In-Vitro-Erfassung und/oder Quantifizierung nach Anspruch 8, umfassend die folgenden Schritte:
(a) **Markieren** der kolorektalen Krebsstammzellen mit einem Lektin, das die Fucose-a 1-2 Galactose-Einheit erkennt, in der biologischen Probe, um markierte kolorektale Krebsstammzellen in der biologischen Probe zu erhalten,
(b) **Sichtbarmachung** des Lektins in der kolorektalen biologischen Probe,
(c) Erfassung und/oder Quantifizierung der durch das Lektin markierten kolorektalen Krebsstammzellen in dem biologischen Gewebe.

10. Verwendung eines Lektins, das die Fucose-a 1-2 Galactose-Einheit erkennt, und eventuell eines Anti-OCT4-Antikörper für die In-Vitro-Diagnose der Rezidivgefahr eines kolorektalen Krebses und/oder der Aggressivität eines kolorektalen Krebses, um den Prognosewert für die therapeutische Adaptation eines kolorektalen Krebses zu definieren.

11. In-Vitro-Diagnoseverfahren der Rezidivgefahr und/oder der Aggressivität eines kolorektalen Krebses, um einen Prognosewert für die therapeutische Adaptation eines kolorektalen Krebses zu definieren, umfassend die folgenden Schritte:
(a3) **Markieren** der kolorektalen Krebsstammzellen mit einem Anti-OCT4-Antikörper in einem histologischen Schnitt eines kolorektalen Gewebes, um kolorektale Krebsstammzellen in dem histologischen Schnitt zu erhalten,
(b3) Inkontaktbringen des in Schritt (a3) erhaltenen histologischen Schnitts mit einem sekundären Antikörper, der den Anti-OCT4-Antikörper erkennt, der an ein Mittel zur Sichtbarmachung C gebunden ist,
(c3) **Sichtbarmachung** des Anti-OCT4-Antikörpers,
(d3) **Markieren** der kolorektalen Krebsstammzellen des in Schritt (c3) erhaltenen histologischen Schnitts mit einem biotinylierten Lektin, das die Fucose-a 1-2 Galactose-Einheit erkennt, und eventuell mindestens einem biotinylierten Lektin, das das T-Antigen erkennt, um kolorektale Krebsstammzellen zu erhalten, die durch das biotinylierte Lektin, das die Fucose-a 1-2 Galactose-Einheit erkennt, und eventuell mindestens ein biotinyliertes Lektin, das das T-Antigen in dem histologischen Schnitt erkennt, markiert sind,
(e3) Inkontaktbringen des in Schritt (d3) erhaltenen histologischen Schnitts mit Streptavidin, Avidin oder einem Anti-Biotin-Antikörper, der an ein Mittel zur Sichtbarmachung A gebunden ist,
(f3) **Sichtbarmachung** des Lektins, das die Fucose-a 1-2 Galactose-Einheit erkennt, und eventuell mindestens eines Lektins, das das T-Antigen erkennt,
(g3) Erfassung und/oder Quantifizierung der kolorektalen Krebsstammzellen in dem in Schritt (f3) erhaltenen histologischen Schnitt,
(h3) Ableitung der Rezidivgefahr und/oder der Aggressivität eines kolorektalen Krebses, um einen Prognosewert für die therapeutische Adaptation eines kolorektalen Krebses aus der Präsenz und/oder der Anzahl von kolorektalen Krebsstammzellen zu definieren.

12. Set, umfassend:
• ein biotinyliertes Lektin, das die Fucose-a 1-2 Galactose-Einheit erkennt, und eventuell mindestens ein biotinyliertes Lektin, das das T-Antigen erkennt, vorteilhafterweise ausgewählt unter ABA, ACA und Jacalin,
• Streptavidin, das an eine Meerrettichperoxidase gebunden ist,
• einen Anti-OCT4-Antikörper,
• einen sekundären Antikörper, der den Anti-OCT4-Antikörper erkennt, der an eine Alkali-Phosphatase gebunden ist.

## Claims

1. Use of a lectin recognizing the fucose α 1-2 galactose group, advantageously UEA-1 or TJA-II, as a first means of labelling of colorectal cancer stem cells, for detecting and/or quantifying said colorectal cancer stem cells.

2. Use according to claim 1, of a lectin recognizing the fucose α 1-2 galactose group and of at least one second means of labelling colorectal cancer stem cells, preferably an anti-OCT4 antibody.

3. Use according to claim 2, of a lectin recognizing the fucose α 1-2 galactose group, of an anti-OCT4 antibody and of at least one lectin recognizing the T-antigen, advantageously chosen from ACA, ABA and Jacaline.

4. Use according to claim 3, in which a means of revealing A of the lectin recognizing the fucose α 1-2 galactose group, advantageously chosen from a chromophore, a fluorophore, and an enzyme capable of reducing a chromogenic substrate B is conjugated with streptavidin, the said lectin recognizing the fucose α 1-2 galactose group being biotinylated.

5. Use according to one of claims 2 to 4, in which a means of revealing **C** of the anti-OCT4 antibody, advantageously chosen from a chromophore, a fluorophore, or an enzyme capable of reducing a chromogenic substrate **D** is bound to a secondary antibody recognizing the anti-OCT4 antibody.

6. Use according to one of claims 1 to 5, in which the colorectal biological sample is a histological section.

7. Use according to one of claims 1 to 6, in which:
• The biological sample is a histological section,
• The lectin recognizing the fucose α 1-2 galactose group is biotinylated UEA-1,
• The means of revealing **A** is a horseradish peroxidase,
• The chromogenic substrate **B** is the diaminobenzidine,
• The means of revealing **C** is an alkalin phosphatase bound to a secondary antibody,
• The chromogenic substrate **D** is the 3-amino-4-methoxybenzamide

8. *In vitro* method of detecting and/or quantifying colorectal cancer stem cells in a colorectal biological sample, comprising a step of revealing a lectin recognizing the fucose α 1-2 galactose group in a colorectal biological sample labeled by the said lectin.

9. *In vitro* method of detecting and/ or quantifying according to claim 8, comprising the steps of:
• **Labelling** colorectal cancer stem cells with a lectin recognizing the fucose α 1-2 galactose group in said biological sample, to obtain labeled colorectal cancer stem cells in said biological sample,
• **Revealing** said lectin in the colorectal biological sample,
• Detecting and/or quantifying the colorectal cancer stem cells labeled by said lectin in said biological tissue.

10. Use of a lectin recognizing the fucose α 1-2 galactose group, and possibly of an anti-OCT4 antibody, for the *in vitro* diagnosis of the risk of recurrence of a colorectal cancer and/or aggressiveness of a colorectal cancer to define the prognostic value for the therapeutic adaptation of a colorectal cancer.

11. *In vitro* method for the diagnosis of the risk of recurrence and/or aggressiveness of a colorectal cancer to define a prognostic value for the therapeutic adaptation of a colorectal cancer comprising the steps of:
• (a3) **labelling** colorectal cancer stem cells with an anti-OCT4 antibody in a histological section of a colorectal tissue, to obtain colorectal cancer stem cells in said histological section,
• (b3) contacting the histological section obtained at step (a3) with a secondary antibody recognizing the anti-OCT4 antibody bound to a means of revealing C,
• (c3) **revealing** the anti-OCT4 antibody,
• (d3) **labelling** colorectal cancer stem cells of the histological section obtained at step (c3) with a biotinylated lectin recognizing the fucose α 1-2 galactose group and possibly at least a biotinylated lectin recognizing the T-antigen, to obtain colorectal cancer stem cells labeled by the biotinylated lectin recognizing the fucose α 1-2 galactose group and possibly at least one biotinylated lectin recognizing the T-antigen in the histological section,
• (e3) contacting the histological section obtained at step (d3) with streptavidin, avidin or an anti-biotin antibody bound to a means of revelation A,
• (f3) **revealing** the lectin recognizing the fucose α 1-2 galactose group and of the possibly at least one lectin recognizing the T-antigen,
• (g3) detecting and/or quantifying colorectal cancer stem cells in the histological section obtained at step (f3),
• (h3) deducing the risk of recurrence and/or aggressiveness of colorectal cancer to define a prognostic value for the therapeutic adaptation of a colorectal cancer from the presence and/or the quantity of colorectal cancer stem cells.

12. Kit comprising:
• A biotinylated lectin recognizing the fucose α 1-2 galactose group and possibly at least one biotinylated lectin recognizing the T-antigen, advantageously chosen from ABA, ACA and Jacaline,
• Streptavidin bound to a horseradish peroxidase,
• An anti-OCT4 antibody,
• A secondary antibody recognizing the anti-OCT4 antibody bound to an alkaline phosphatase.
